(19) Europäisches Patentamt
European Patent Office
Office européen des brevets

(11) **EP 3 439 618 B1**

(12) **EUROPEAN PATENT SPECIFICATION**

(45) Date of publication and mention
of the grant of the patent:
**01.12.2021 Bulletin 2021/48**

(21) Application number: **17720224.9**

(22) Date of filing: **06.04.2017**

(51) Int Cl.:
$A61K\ 8/29^{(2006.01)}$     $A61Q\ 17/04^{(2006.01)}$
$A61K\ 8/81^{(2006.01)}$     $A61K\ 8/02^{(2006.01)}$
$A61K\ 8/19^{(2006.01)}$

(86) International application number:
**PCT/IB2017/051975**

(87) International publication number:
**WO 2017/175164 (12.10.2017 Gazette 2017/41)**

(54) **UV-PROTECTIVE COMPOSITIONS AND THEIR USE**

UV-SCHUTZZUSAMMENSETZUNGEN UND IHRE VERWENDUNG

COMPOSITIONS ANTI-UV ET LEUR UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **06.04.2016 GB 201605857**

(43) Date of publication of application:
**13.02.2019 Bulletin 2019/07**

(73) Proprietor: **Landa Labs (2012) Ltd.**
**7612301 Rehovot (IL)**

(72) Inventors:
• **LANDA, Benzion**
**7405135 Nes Ziona (IL)**
• **ABRAMOVICH, Sagi**
**4339320 Ra'anana (IL)**
• **DOR, Snir**
**4906437 Petach Tikva (IL)**

(74) Representative: **Harrison IP Limited**
**3 Ebor House**
**Millfield Lane**
**Nether Poppleton, York YO26 6QY (GB)**

(56) References cited:
**EP-A1- 2 229 931**     **CN-A- 1 804 152**
**CN-A- 102 963 929**

• **JUN YOUNG HAN ET AL: "Influence of transition
metal doping (X?=?Co, Fe) on structural, optical
properties of Ferroelectric
Bi3.25La0.75X1Ti2O12", NANO CONVERGENCE,
vol. 2, no. 1, 8 April 2015 (2015-04-08),
XP55385483, DOI: 10.1186/s40580-014-0035-1**
• **HUA KE ET AL: "Crystallization process of
lanthanum-substituted bismuth titanate
synthesized by a facile sol-gel method",
JOURNAL OF SOL-GEL SCIENCE AND
TECHNOLOGY, KLUWER ACADEMIC
PUBLISHERS, BO, vol. 53, no. 1, 10 September
2009 (2009-09-10), pages 135-140, XP019772183,
ISSN: 1573-4846**

**Description**

**FIELD**

The present disclosure relates to the field of protection from ultraviolet radiation, and more particularly, to UV protective compositions comprising Lanthanum-Modified Bismuth Titanate (BLT) crystals, neat or polymer-embedded, to methods for preparing the same and uses thereof.

**BACKGROUND**

Ultraviolet (UV) radiation is ubiquitous, the sun being the most common source of UV radiation although not the only source. As UV radiation can cause damage to people, animals and objects, compositions that provide protection from UV radiation are useful.

In the biological context, UV-protective compositions, *i.e.* compositions that reduce or block the transmission of UV rays, are commonly employed to protect against sunburn. Sunburn is a form of radiation burn resulting from an overexposure to UV radiation, typically from the sun, but also from artificial sources, such as tanning lamps, welding arcs, and ultraviolet germicidal irradiation.

Normal symptoms of sunburn in humans and other animals include reddening of the skin, general fatigue and mild dizziness. An excess of UV radiation can be life-threatening in extreme cases. Excessive UV radiation is considered to be the leading cause of non-malignant skin tumors, as well as increasing the risk of certain types of skin cancer.

Sunscreen compositions comprising UV protective agents are commonly used to prevent sunburn and are believed to prevent squamous cell carcinomas and melanomas. Furthermore, they have been reported to delay the development of wrinkles and additional age-related skin conditions.

Specifically, sunscreen compositions are topical compositions that include UV-protecting agents that absorb and/or reflect at least some of the sun's UV radiation on areas of skin exposed to sunlight, and thus reduce the effect of UV radiation on the skin. Depending on their mode of action, they are typically classified as chemical or physical sunscreens.

Chemical sunscreen compositions comprise organic compounds that absorb UV radiation to reduce the amount of UV radiation that reaches the skin. Being transparent to visible light and thereby being invisible when applied to the skin, chemical sunscreen compositions are popular for use. However, some organic compounds used in chemical sunscreen compositions have been found to generate free radicals which can cause skin damage, irritation and accelerated aging of the skin. Furthermore, organic materials may be absorbed into the skin, resulting in long-term detrimental health effects. Chemical sunscreen compositions may require the addition of a photostabilizer.

Physical sunscreen compositions reflect and absorb UV radiation. Known physical sunscreen compositions comprise particles of inorganic materials, mainly titanium oxide and/or zinc oxide. In order to obtain absorption and/or reflection of ultraviolet radiation over the full UVA and UVB range, relatively large particles are used. Due to the large particle size, such sunscreen compositions are viscous and opaque and tend to leave a white cast on the skin.

Many sunscreen compositions protect against UV radiation in the 280-315 nm range (UVB radiation) that causes sunburn, but do not against UV radiation in the 315-400 nm range (UVA radiation) which does not primarily cause sunburn but can increase the rate of melanoma and photodermatitis.

It is generally preferred that sunscreen compositions, when applied to the skin, are transparent to the eye. In order for physical sunscreen compositions to be transparent, the particles of inorganic material should be in the form of nanoparticles, which absorb and/or scatter UV light but not visible light, rendering them substantially transparent to the eye when applied to the skin. However, use of nanoparticles reduces the range of wavelengths absorbed by the inorganic materials. Some known sunscreen compositions therefore block both UVA and UVB radiation by use of a combination of different UV-absorbing or scattering materials, generally termed UV-protecting agents, each of which blocks radiation over a limited range of the UV spectrum.

Similarly, UV-protective compositions can benefit inert materials or objects that may be negatively affected by UV radiation. For instance, UV radiation can reduce the life-span of materials (e.g., natural and synthetic polymers), and may modify colors of objects, especially in articles that are subjected to prolonged sun exposure, such as buildings or vehicles.

Various coatings are known to provide protection against UV radiation damage by blocking or reducing transmission of UV rays. Use of such coatings may in turn reduce the detrimental effect of UV radiation on a living animal. For example, use of said coating on optical lenses, thereby reducing the transmission of UV radiation, may reduce the incidence of UV-induced optical disorders such as cataract. Materials serving for the fabrication of windows incorporating or coated with suitable UV-protecting agents may reduce the transmission of UV radiation to subjects, plants, surfaces or objects shielded by such windows.

The present Applicant has disclosed sunscreen compositions comprising inorganic nanoparticles, *inter alia* in PCT Publication Nos. WO 2016/151537 and WO 2017/013633.

It would be desirable to have an effective UV protective composition, in particular providing broad-spectrum protection, and safe for use on living subjects.

## SUMMARY

The present disclosure, in at least some embodiments thereof, provides ultraviolet radiation protective compositions, such as, sunscreen compositions, that when applied to a surface provides protection from UV radiation, which in some embodiments have a broad-spectrum UV protective activity, such compositions comprising Lanthanum-Modified Bismuth Titanate (BLT) crystals, doped by iron atoms.

According to an aspect of some embodiments, there is provided a UV-protective composition comprising one or more Lanthanum-Modified Bismuth Titanate (BLT) crystals each independently having the chemical formula $Bi_{(4-x)}La_{(x)}Ti_{(3-y)}Fe_{(y)}O_{12}$ as an ultraviolet-absorbing agent, wherein x is between 0.1 and 1.5; and wherein y is between <0 and 2, according to claim 1.

The doped (*i.e.,* y>0) BLT crystals are a composite material, having properties which differ from those individually characterizing their constituting starting compounds. One or more crystals, of the same or different general chemical formula, may form particles or nanoparticles as described below.

The Lanthanum-Modified Bismuth Titanate crystals can be synthesized using different ratios of Bismuth Trioxide ($Bi_2O_3$; also referred to as Bismuth(III) Oxide or simply Bismuth Oxide), Titanium Dioxide ($TiO_2$; often referred to as Titanate or Titanium Oxide) and Lanthanum Oxide ($La_2O_3$) by a variety of methods readily known to the person skilled in the art of preparing such composite materials.

For conciseness, the mixture of the individual metal oxide constituents shall be referred to as BLTO, whereas the crystal as prepared, comprising the composite material, shall be termed hereinafter BLT, such acronyms eventually followed by the ratio between at least two of the constituents. The ratio is typically provided on a molar basis, but may also be provided on a weight per weight basis. As used herein, the term "BLT" includes both the doped and the undoped crystal.

In the event that iron atoms (as available for instance from Iron(III) Oxide or Ferric Oxide ($Fe_2O_3$)) optionally substitute atoms of the composite material, typically Titanium, the so-called "doped" crystal is formed. In such case, the crystal acronym for the chemical formula may eventually be followed by the molar ratio of substitution between the iron substituent and the atom being replaced. For instance, BLT Fe:Ti 1:2 refers to a Lanthanum-Modified Bismuth Titanate composite material wherein 1 mole of Ferric Oxide ($Fe_2O_3$) is included in the synthetic process for every 2 moles of Titanium Oxide ($TiO_2$). BLTO Fe:Ti 1:2 refers to same amounts of metal oxide constituents, including the Ferric Oxide intended for substitution, however the compounds are only mixed and not further processed for the preparation of the previously described composite material and resulting crystal.

According to an aspect of some embodiments, there is provided a UV-protective composition comprising one or more Lanthanum-Modified Bismuth Titanate (BLT) crystals each independently having the chemical formula $Bi_{(4-x)}La_{(x)}Ti_{(3-y)}Fe_{(y)}O_{12}$ as an ultraviolet-absorbing agent, wherein x is between 0.1 and 1.5; and wherein y is between <0 and 2.

In some embodiments, the doped BLT crystals have a perovskite structure.

In some embodiments, x is between 0.5 and 1.0, such as between 0.7 and 0.8.

In some embodiments, y is between 0.01 and 1.8. The BLT crystal wherein y is greater than zero can also be referred to as a doped or an Fe-doped BLT crystal.

In some embodiments, the molar ratio of Fe to Ti is selected from (0.0625 and 2.9375), (0.125 and 2.875), (0.25 and 2.75), (1 and 2) and (1.5 and 1.5).

The compositions described herein are for use in both living subjects and inanimate objects (e.g., UV protective coating of articles routinely exposed to UV radiation).

Therefore, some embodiments of the present disclosure relate to compositions providing protection against ultraviolet radiation (*i.e.* UV protective compositions), and more particularly, to UV protective compositions comprising BLT crystals, optionally doped by iron atoms, as an ultraviolet-absorbing agent.

The doped BLT crystals are present in the composition as nanoparticles consisting of one or more said crystals, at least 50% of the total number of said nanoparticles having at least one dimension of up to about 200 nm, in some embodiments up to about 150 nm or up to about 100 nm. In some such embodiments, the nanoparticles consist of crystals having the same chemical formula

In some embodiments, the doped BLT crystals are present in the composition as nanoparticles consisting of one or more said crystals, at least 50% of the total volume of said nanoparticles having at least one dimension of up to about 200 nm, in some embodiments up to about 150 nm or up to about 100 nm.

In some embodiments, at least 55%, at least 60%, at least 65%, at least 70%, at least 80%, or at least 85% of the total number or total volume of nanoparticles has at least one dimension of up to about 200 nm, in some embodiments up to about 150 nm or up to about 100 nm. In some such embodiments, the nanoparticles consist of crystals having the same chemical formula.

In some embodiments, at least 90%, or at least 95%, or at least 97.5%, or at least 99% of the total number or total volume of nanoparticles of the doped BLT crystals present in the composition has a hydrodynamic diameter of up to about 200 nm, or up to 150 nm, or up to about 100 nm.

In some embodiments, the doped BLT nanoparticles are present in the composition dispersed in a polymer matrix. In particular embodiments the nanoparticles of the composite UV-absorbing agent are dispersed in the polymer matrix in presence of a dispersant, the polymer matrix being in an oil-based carrier.

In some embodiments, the composition contains less than 5 weight per weight percentage (wt.%), less than 4 wt.%, less than 3 wt.%, less than 2 wt.%, less than 1 wt.%, less than 0.5 wt.%, less than 0.1 wt.% or less than 0.05 wt.% organic ultraviolet-absorbing agent(s). Suitably the composition is generally devoid of an organic ultraviolet-absorbing agent. Typically, the composition is free of an organic ultraviolet-absorbing agent.

In some embodiments, the composition contains less than 5 wt.%, less than 4 wt.%, less than 3 wt.%, less than 2 wt.%, less than 1 wt.%, less than 0.5 wt.%, less than 0.1 wt.% or less than 0.05 wt.% additional inorganic ultraviolet-absorbing agent(s). Suitably the composition is generally devoid of an additional inorganic ultraviolet-absorbing agent. Typically, the composition is free of an additional inorganic ultraviolet-absorbing agent. In some embodiments, the one or more doped BLT crystals constitute the only ultraviolet-absorbing agents in the composition.

In some embodiments, the doped BLT crystals are present in the composition in the form of nanoparticles at a concentration in the range of from about 0.001 wt.% to about 40 wt.% of the total composition.

In some embodiments, the composition further comprises silver particles.

In some embodiments, the silver particles comprise silver nanoparticles having at least one dimension of up to about 50 nm.

In some embodiments, at least 90%, at least 95%, at least 97.5% or at least 99% of the number of silver nanoparticles present in the composition has at least one dimension of up to about 50 nm.

In some embodiments, at least 90%, at least 95%, at least 97.5% or at least 99% of the volume of silver nanoparticles present in the composition has at least one dimension of up to about 50 nm. In some embodiments, wherein the composition comprises silver nanoparticles, the composition is devoid of an additional ultraviolet-absorbing agent.

In some embodiments, the silver particles are present in the composition at a concentration in the range of from about 0.01 wt.% to about 10 wt.% of the total composition.

In some embodiments, the composition further comprises one or more of a carrier, an excipient, an additive and combinations thereof. Carriers, excipients and additives being cosmetically acceptable are preferred for use in living subjects, but may not be required for use on the surfaces of inanimate objects.

In some embodiments, the composition is in a form selected from the group consisting of an aerosol, a cream, an emulsion, a gel, a lotion, a mousse, a paste, a liquid coat or a spray.

In some embodiments, the composition is formulated as one of the following: (a) a skin-care composition for application to human or non-human animal skin; (b) a hair-care composition for application to human or non-human animal hair; or (c) a coating composition for application to an inanimate surface.

In a further aspect, embodiments of the present disclosure provide use of afore-described doped BLT crystals for the preparation of a composition for protecting a target surface, such as a surface of a living subject and/or an inanimate object, against a harmful effect of UV radiation. The compositions, comprising an efficacious amount of BLT, can be formulated as suitable for application upon the intended surfaces, such preparations being known to persons skilled in the relevant formulations.

According to one embodiment, there is provided a composition as described herein, for use in protecting a subject against a harmful effect of UV radiation

According to one embodiment, there is provided a composition as described herein, for use in protecting the skin of a subject against a harmful effect of UV radiation. In some such embodiments, the composition is in the form of a topical composition. In such embodiments, the composition can be in any form suitable to skin-care products, such as facial-care products, make-up products, body-care products, hand-care products and/or foot-care products. Such skin-care products can be applied to the skin of a subject by any conventional method and/or for any duration of time that need not be detailed herein.

According to a further embodiment, there is provided a composition as described herein, for use in protecting the hair of a subject against a harmful effect of UV radiation. In some such embodiments, the composition is in the form of a hair-care product, such as a hair-care product selected from the group consisting of a shampoo, a conditioner and a hair mask. Such hair-care products can be applied to the hair of a subject by any conventional method and/or for any duration of time that need not be detailed herein.

In some embodiments of a use of the composition, the subject is a human subject. In alternative embodiments of a use of the composition, the subject is a non-human animal.

In some embodiments of the use of the composition, the target surface is a surface of an inanimate object, such as, for example, an object, or a material. In some such embodiments, the composition is in the form of a coating, including liquid coatings, such as a varnish, a lacquer or an emulsion, and non-liquid coatings, such as a paste, a gel, or a mousse. Though UV-protective compositions applicable to the surfaces of inanimate objects are herein referred to as "coatings", it will be readily understood that such compositions may also permeate, impregnate or be otherwise embedded at least to some extent within the surfaces of the objects being protected. Such coating products can be applied to the surface of an inanimate object by any conventional method that need not be detailed herein.

In some embodiments, protecting against ultraviolet radiation comprises protecting against a harmful effect of ultraviolet A radiation and ultraviolet B radiation.

In some embodiments, the composition has a critical wavelength of at least 370 nm, such as 371 nm, 372 nm, 373 nm, 374 nm, 375 nm, 376 nm, 377 nm, 378 nm, 379 nm, 380 nm, 381 nm, 382 nm, 383 nm, 384 nm, 385 nm, 386 nm, 387 nm, 388 nm, 389 nm, 390 nm, 391 nm, 392 nm, or greater than 392 nm.

In some embodiments, the area under the curve (AUC) formed by the UV-absorption of the one or more BLT crystals as a function of wavelength in the range of 280 nm to 400 nm ($AUC_{280-400}$) is at least 75%, at least 85% or at least 95% of the AUC formed by the same crystals at the same concentration in the range of 280 nm to 700 nm ($AUC_{280-700}$).

According to a further aspect of some embodiments of the disclosure, there is provided a method of manufacturing a UV protective composition, comprising combining doped BLT crystals, as an ultraviolet-absorbing agent, with other ingredients in proportions and in a manner suitable to make a UV-protective composition as described herein. In some embodiments, the UV-protective composition is manufactured and formulated as a sunscreen composition for application to skin or hair of a human or non-human living subject. In some embodiments, the composition is manufactured and formulated as a composition for application to a surface of an inanimate object.

There is also provided, in accordance with an embodiment of the invention, a method of protecting a surface from UV radiation, which comprises applying to a surface in need of such protection a UV-protective composition as described herein in an amount sufficient to achieve such protection. In some embodiments, the surface is human skin. In some embodiments, the surface is non-human skin, *i.e.* animal skin. In some embodiments, the surface is hair. In some embodiments, the hair is human hair. In some embodiments, the hair is non-human hair, *i.e.* animal hair. In some embodiments, the surface is a surface of an inanimate object.

As used herein, the term "nanoparticles" refers to particles of any suitable shape, which may consist of one or more crystals as herein disclosed, wherein the size of at least one dimension is 200 nm or less, hereinafter also referred to as the smallest dimension, and wherein a greatest size in a different dimension of the particles, also termed a greatest dimension, is of no more than about 500 nm.

For example, in some embodiments where the particles have a flake-like shape, the smallest dimension of the nanoparticles can be their thickness which can be of up to about 200 nm, while their length can be of no more than about 500 nm.

For example, in some embodiments where the particles have a rod-like shape, their cross section along their longitudinal axis could be approximated to ellipsoids having at least their minor axis constituting a smallest dimension of no more than about 200 nm and the length of the rods being no more than about 500 nm.

For example, in some embodiments where the particles have a sphere-like shape that could be approximated by three diameters one for each of the X-, Y- and Z-direction, at least one of the three diameters is not more than about 200 nm and a greatest of the three diameters can be no more than about 500 nm.

In some embodiments, the smallest dimension of the nanoparticles is not more than about 180 nm, not more than about 160 nm, not more than about 140 nm, not more than about 120 nm, or not more than about 100 nm.

In some embodiments, the smallest dimension of the nanoparticles is at least about 10 nm, at least about 15 nm or at least about 20 nm.

In some embodiments, the greatest dimension of the nanoparticles is not more than about 400 nm, not more than about 300 nm, not more than about 200 nm, or not more than about 150 nm.

In some embodiments, the nanoparticles of BLT and/or the compositions including the BLT crystals disclosed herein are substantially invisible to the human eye, in particular when applied to a subject.

In some embodiments, the compositions are visible to the human eye when applied to a subject. In some such embodiments, iron doped BLT provides a reddish colour that is beneficial in the preparation of a product in which such colour is desirable, *e.g.* a make-up product such as a blusher, or a tinted coating for application to a surface of an inanimate obj ect.

In some embodiments, the size of the particles (e.g., BLT nanoparticles or matrix elements or flakes optionally embedding them) is determined by microscopy techniques, as known in the art.

In some embodiments, the size of the particles is determined by Dynamic Light Scattering (DLS). In DLS techniques the particles are approximated to spheres of equivalent behavior and the size can be provided in term of hydrodynamic diameter. DLS also allows assessing the size distribution of a population of particles.

Distribution results can be expressed in terms of the hydrodynamic diameter for a given percentage of the cumulative particle size distribution, either in terms of numbers of particles or volumes, and are typically provided for 10%, 50% and 90% of the cumulative particle size distribution. For instance, D50 refers to the maximum hydrodynamic diameter below which 50% of the sample volume or number of particles, as the case may be, exists and is interchangeably termed the median diameter per volume ($D_V50$) or per number ($D_N50$), respectively.

In some embodiments, the nanoparticles of the disclosure have a cumulative particle size distribution of D90 of 200 nm or less, or a D95 of 200 nm or less, or a D97.5 of 200 nm or less or a D99 of 200 nm or less, *i.e.* 90%, 95%, 97.5% or 99% of the sample volume or number of particles respectively, have a hydrodynamic diameter of no greater than 200 nm.

In some embodiments, the cumulative particle size distribution of the population of nanoparticles is assessed in term of number of particles (denoted $D_N$) or in term of volume of the sample (denoted $D_V$) comprising particles having a given hydrodynamic diameter.

Any hydrodynamic diameter having a cumulative particle size distribution of 90% or 95% or 97.5% or 99% of the particles population, whether in terms of number of particles or volume of sample, may be referred to hereinafter as the "maximum diameter", *i.e.* the maximum hydrodynamic diameter of particles present in the population at the respective cumulative size distribution.

It is to be understood that the term "maximum diameter" is not intended to limit the scope of the present teachings to nanoparticles having a perfect spherical shape. This term as used herein encompasses any representative dimension of the particles at cumulative particle size distribution of at least 90%, *e.g.,* 90%, 95%, 97.5% or 99%, or any other intermediate value, of the distribution of the population.

Dimensions of particles can also be assessed (or confirmed) by microscopy (*e.g.*, light microscopy, confocal microscopy, SEM, STEM, *etc.*). Such techniques are deemed more suitable than DLS for particles (such as matrix flakes) having non-globular shapes. The particles may be characterized by an aspect ratio, *e.g.,* a dimensionless ratio between the smallest dimension of the particle and the longest dimension or equivalent diameter in the largest plane orthogonal to the smallest dimension, as relevant to their shape. The equivalent diameter (Deq) is defined by the arithmetical average between the longest and shortest dimensions of that largest orthogonal plane. Particles having an almost spherical shape are characterized by an aspect ratio of approximately 1:1, whereas flake-like particles, such as matrix flakes, can have an aspect ratio of up to 1:100, or even more.

As further detailed herein-below, nanoparticles of BLT crystals can in some embodiments be immobilised within a polymer matrix. The matrix can form distinct elements, which may assume a variety of shapes. For topical application, a platelet shape is deemed particularly suitable. Such matrix flakes can be characterized by a flake length (Lf, the longest dimension in the plane of the flake), a flake width (Wf, the largest dimension in the plane of the flake, such width being orthogonal to the length), and a flake thickness (Tf, the largest thickness being measured orthogonally to the plane in which the length and width of the flake are defined). Lf, Wf and Tf can be further used to calculate an aspect ratio (*e.g.,* Rf as below defined) of a matrix flake.

Such characteristic dimensions can be assessed on a number of representative particles, or a group of representative particles, that may accurately characterize the population (*e.g.*, by diameter, longest dimension, thickness, aspect ratio and like characterizing measures of the particles). It will be appreciated that a more statistical approach may be desired for such assessments. When using microscopy for particle size characterization, a field of view of the image-capturing instrument (*e.g.,* light microscope, *etc.*) is analyzed in its entirety. Typically, the magnification is adjusted such that at least 5 particles, at least 10 particles, at least 20 particles, or at least 50 particles are disposed within a single field of view. Naturally, the field of view should be a representative field of view as assessed by one skilled in the art of microscopic analysis. The average value characterizing such a group of particles in such a field of view is obtained by volume averaging. In such case, $D_V50 = \sum[(Deq(m))^3/m]^{1/3}$, wherein *m* represents the number of particles in the field of view and the summation is performed over all *m* particles. As mentioned, when such methods are the technique of choice for the scale of the particles to be studied or in view of their shape, such measurements can be referred to as D50.

As used herein, the terms "ultraviolet-protective agent" or "ultraviolet-protecting agent" refer to agents that absorb and/or reflect and/or scatter at least some of the UV radiation on surfaces exposed to sunlight or any other UV source, and thus reduce the effect of UV radiation on the surface. The surface may be the skin and/or hair of a subject, such as a human subject. The surface may also be the surface (*e.g.,* an exterior face) of an inanimate obj ect.

In another aspect, embodiments of the present disclosure provide a method for the preparation of afore-described compositions.

Some known UV protective compositions block both UVA and UVB radiation by use of a combination of different UV-protecting agents, each of which blocks radiation over a limited range of the UV spectrum.

As used herein, the term "broad-spectrum UV absorption" with regard to an ultraviolet-absorbing agent refers to an ultraviolet-absorbing agent that absorbs both UVA and UVB radiation. In some embodiments, the breadth of UV absorption may be measured according to the Critical Wavelength Method, wherein an ultraviolet-absorbing agent is considered to provide broad spectrum absorption when the critical wavelength is greater than 370 nm, and unless otherwise noted, in the present disclosure the term "broad-spectrum UV absorption" as used herein is determined on the basis of the critical wavelength.

As used herein, the term "critical wavelength" is defined as the wavelength at which the area under the absorbance spectrum from 290 nm to the critical wavelength constitutes 90% of the integral of the absorbance spectrum in the range from 290 nm to 400 nm.

In some instances, noted as such herein, the term "broad-spectrum UV absorption" with regard to an ultraviolet-absorbing agent refers to the situation in which the area under the curve (AUC) formed by the UV-absorption of the agent as a function of wavelength in the range of 280 nm to 400 nm ($AUC_{280-400}$) is at least 75% of the AUC formed by the same agent at the same concentration in the range of 280 nm to 700 nm ($AUC_{280-700}$). Similarly, where noted as such herein, the terms "broader-spectrum UV absorption" and "broadest spectrum UV absorption" with respect to a UV-absorbing agent refer respectively to the situation in which the area under the curve (AUC) formed by the absorption of the agent as a function of wavelength in the range of 280 nm to 400 nm ($AUC_{280-400}$) is at least 85% or 95% of the AUC formed by the same agent at the same concentration in the range of 280 nm to 700 nm ($AUC_{280-700}$).

As used herein, the term "ultraviolet-absorbing agent" refers to an agent which, when present in a composition at up to 50 wt.% of the total composition, provides at least 50% absorption of ultraviolet light in the wavelength range of from 290 nm to 400 nm.

As used herein, the terms "generally devoid of an organic ultraviolet-absorbing agent", "considerably devoid of an organic ultraviolet-absorbing agent", "significantly devoid of an organic ultraviolet-absorbing agent", "substantially devoid of an organic ultraviolet-absorbing agent", "essentially devoid of an organic ultraviolet-absorbing agent", "substantively devoid of an organic ultraviolet-absorbing agent" and "devoid of an organic ultraviolet-absorbing agent" refer respectively to a composition in which a UV-absorbing organic material, if any, is present in the composition at a concentration which provides absorption of not more than 20%, not more than 15%, not more than 10%, not more than 5%, not more than 2%, not more than 1% or not more than 0.5% of ultraviolet light in the wavelength range of from 290 nm to 400 nm.

As used herein, the term "generally devoid of an additional ultraviolet-absorbing agent", "considerably devoid of an additional ultraviolet-absorbing agent", "significantly devoid of an additional ultraviolet-absorbing agent", "substantially devoid of an additional ultraviolet-absorbing agent", "essentially devoid of an additional ultraviolet-absorbing agent", "substantively devoid of an additional ultraviolet-absorbing agent" and "devoid of an additional ultraviolet-absorbing agent" refer respectively to a composition which is devoid of any UV-absorbing material other than that specifically disclosed as being present in the composition at a concentration, which, if included in the composition, provides absorption of not more than 20%, not more than 15%, not more than 10%, not more than 5%, not more than 2%, not more than 1% or not more than 0.5% of ultraviolet light in the wavelength range of from 290 nm to 400 nm.

According to an aspect of some embodiments, the present disclosure relates to compositions providing protection against ultraviolet radiation, and more particularly, to UV protective compositions comprising a matrix comprising a polymer and an oil, and doped BLT crystals and a dispersant, wherein the crystals are dispersed in the matrix. Advantageously, the dispersed crystals do not substantially migrate out of the polymer matrix. In such case, the crystals may also be said to be immobilised or embedded in the matrix, also referred to as the polymer matrix or the swelled polymer matrix.

According to an aspect of some embodiments of the disclosure, there is provided a matrix comprising a polymer and an oil; and doped BLT crystals and a dispersant, dispersed in the matrix.

In some embodiments, the doped BLT crystals are present in said matrix at a concentration of from about 0.1 wt.% to about 60 wt.% of the polymer, or from about 3 wt.% to about 40 wt.%, optionally at a concentration of about 5 wt.% to 20 wt.% of the polymer of the matrix.

In some embodiments, the doped BLT crystals are present in said matrix at a concentration of from about 0.01 to about 8% (volume per volume or v/v) of the polymer, or from about 0.4 to about 5% (v/v), optionally at a concentration of about 0.6 to about 3% (v/v) of the polymer of the matrix.

In some embodiments, the doped BLT crystals are present in the matrix at a concentration of from about 1 to about 10% (weight per weight, w/w or wt.%) or from about 1 to about 10% (v/v) of the total composition, optionally at a concentration of about 4% (w/w) or 0.5% (v/v) of the composition.

In some embodiments, the oil is present in the polymer matrix at a concentration of from about 10 to about 50% (w/w) of the polymer of the matrix or from about 5 to about 50% (v/v) of the polymer of the matrix, optionally at a concentration of about 30% (w/w) or about 20% (v/v) of the polymer of the matrix.

In some embodiments, the oil of the matrix is selected from the group consisting of mineral oil, natural oil, vegetal oil, synthetic oil, and combinations thereof.

In some embodiments, the polymer of the matrix is an oil-swellable thermoplastic homo- or co- polymer, optionally clear, transparent and/or colorless.

In some preferred embodiments, the polymers suitable for the matrix are functionalized polymers or copolymers comprising particle-affinic functional group and non-affinic monomer units. For instance, the functional groups may be acidic monomers, whereas the non-affinic groups can be ethylene. In some embodiments, the polymer comprises at least one ethylene-acrylic (EAA) polymer, ethylene-methacrylic (EMMA) polymer, ethyl vinyl acetate (EVA) polymer, and combinations thereof.

In some embodiments, the polymer of the matrix comprises at least one ethylene-acrylic polymer, optionally wherein the ethylene-acrylic polymer comprises from about 5 wt.% to about 30 wt.% acrylic monomer. In some embodiments, the ethylene-acrylic polymer is selected from the group consisting of ethylene-methacrylic acid copolymer and ethylene-acrylic acid copolymer.

In some embodiments, the polymer of the matrix, which can be a copolymer or a combination thereof, have at least one of a softening point and a melting point not exceeding 200°C, said softening point or melting point optionally being of at least 60°C.

The oil and the polymer of the polymer matrix, or a combination of oils and/or a combination of polymers forming such a matrix, are selected and adapted to be compatible one with the other. In other words the oil(s) can swell the polymer(s) and the polymer(s) can be swelled by the oil(s). Swelling (and grammatical variants) refers to the ability of the oil to penetrate a polymeric network formed by the polymer (the matrix), resulting, among other things, in an increase in the weight of the matrix, and typically additionally in an expansion of its volume.

In some embodiments, the matrix is present in the form of matrix elements, at least 50% of the number of matrix elements having at least one dimension of up to about 50 μm, at most 25 μm, at most 10 μm or at most 5 μm.

In some embodiments, the matrix elements of the polymer matrix (e.g., comprising a thermoplastic polymer swelled with an oil and nanoparticles of doped BLT crystals dispersed and embedded therein with a dispersant) are matrix flakes, wherein each flake of the swelled polymer matrix flakes has a flake length (Lf), a flake width (Wf), and a flake thickness (Tf), the matrix flakes having a dimensionless flake aspect ratio (Rf) defined by:

$$Rf = (Lf \cdot Wf)/(Tf)^2$$

wherein, with respect to a representative group of the swelled polymer matrix flakes, an average Rf is at least 5.

In some embodiments, at least one of the flake length (Lf) and the flake width (Wf) of the matrix flakes is at most 50 μm, at most 25 μm, at most 10 μm, or at most 5 μm.

In some embodiments, the flake thickness (Tf) of the matrix flakes is at most 1000 nm, at most 900 nm, at most 750 nm, at most 650 nm, at most 600 nm, at most 550 nm, at most 500 nm, at most 450 nm, at most 400 nm, at most 350 nm, at most 300 nm, or at most 250 nm.

In some embodiments, flake aspect ratio (Rf) of the matrix flakes is within a range of from about 5 to about 2000, from about 10 to about 1000, from about 12 to about 500, from about 12 to about 200, or from about 15 to about 100.

In some embodiments, the representative group is disposed in an instrumental field of view containing at least 10 of the matrix flakes or swelled polymer matrix flakes, and optionally hundreds of nanoparticles of doped BLT crystals.

In some embodiments, at least 50%, at least 60%, at least 75%, or at least 90% of the nanoparticles embedded in the matrix elements or matrix flakes have a cumulative particle size (D50, D60, D75, and D90, accordingly) of at most 100 nm, at most 90 nm, at most 80 nm, at most 70 nm, or at most 60 nm. The cumulative particle size can be determined in terms of percent number of nanoparticles in the population of the plurality of particles or in terms of percent volume. Thus, in some embodiments, the nanoparticles of BLT crystals embedded in the matrix flakes can be characterized by a $D_N50$ of at most 100 nm (up to a $D_N90$ of at most 60 nm) or by a $D_V50$ of at most 100 nm (up to a $D_V90$ of at most 60 nm).

In some embodiments, the dispersant adapted to disperse the nanoparticles of doped BLT crystals within the polymer matrix has a hydrophilic-lipophilic balance (HLB) value of at most 9, at most 6, at most 4, or at most 3.

In some embodiments, the UV-protective composition provides protection against UV radiation selected from the group consisting of a UVA-radiation and a UVB-radiation. In some embodiments, the UV-protective composition provides UVA and UVB protective activity.

Aspects and embodiments of the disclosure are described in the specification herein below and in the appended claims.

Unless otherwise defined, all technical and scientific terms used herein have the same meaning as commonly understood by one of ordinary skill in the art to which the particular teachings pertain. In case of conflict, the specification, including definitions, will take precedence.

As used herein, the terms "comprising", "including", "having" and grammatical variants thereof are to be taken as specifying the stated features, integers, steps or components, but do not preclude the addition of one or more additional features, integers, steps, components or groups thereof. These terms encompass the terms "consisting of' and "consisting essentially of'.

As used herein, the indefinite articles "a" and "an" and the singular form "the" include plural references and mean "at least one" or "one or more" unless the context clearly dictates otherwise.

Unless otherwise stated, the use of the expression "and/or" between the last two members of a list of options for selection indicates that a selection of one or more of the listed options is appropriate and may be made.

In the discussion, unless otherwise stated, adjectives such as "substantially" and "about" that modify a condition or relationship characteristic of a feature or features of an embodiment of the present technology, are to be understood to mean that the condition or characteristic is defined within tolerances that are acceptable for operation of the embodiment for an application for which it is intended, or within variations expected from the measurement being performed and/or from the measuring instrument being used. In particular, when a numerical value is preceded by the term "about", the term "about" is intended to indicate +/-10%, or +/-5%, or +/-2% of the mentioned value and in some instances the precise value.

Additional objects, features and advantages of the present teachings, and aspects of embodiments of the invention, will be set forth in the detailed description which follows, and in part will be readily apparent to those skilled in the art from the description or recognized by practicing embodiments of the invention as described in the written description and claims hereof, as well as the appended drawings. Various features and sub-combinations of embodiments of the present disclosure may be employed without reference to other features and sub-combinations.

## BRIEF DESCRIPTION OF THE DRAWINGS

Some embodiments of the invention are described herein with reference to the accompanying figures. The description, together with the figures, makes apparent to a person having ordinary skill in the art how some embodiments of the disclosure may be practiced. The figures are for the purpose of illustrative discussion and no attempt is made to show structural details of an embodiment in more detail than is necessary for a fundamental understanding of the disclosure. For the sake of clarity, some objects depicted in the figures are not to scale.

In the Figures:

Figure 1 is a plot showing the powder X ray diffraction (PXRD) diffractogram of Fe-doped and undoped BLT crystals prepared according to the present teachings.

Figure 2 is a line graph showing powder absorbance of Fe-doped and undoped BLT crystals prepared according to present teachings, as compared to the mixtures of their respective constituents, BLTO-Fe and BLTO.

Figure 3 is a line graph showing powder absorbance of BLT crystals doped with various ratios of iron to titanium atoms as prepared according to present teachings, as compared to undoped BLT crystals as reference.

Figure 4 is a line graph showing Particle Size Distribution (PSD) of particles of Fe-doped and undoped BLT crystals in aqueous dispersions after milling according to present teachings, expressed as number percentage.

Figure 5 is a line graph showing absorbance of aqueous suspensions comprising different concentrations of nanoparticles of undoped BLT crystals prepared according to present teachings, as compared to a commercial sample and a control consisting of nanoparticles of Zinc Oxide.

Figure 6 is a line graph showing absorbance of aqueous suspensions comprising same concentration of nanoparticles of BLT crystals at various levels of Fe-doping prepared according to present teachings, as compared to undoped BLT.

Figure 7 is a Scanning Transmitting Electron Microscopy (STEM) image captured using a high-resolution Scanning Electron Microscope (HR-SEM) of nanoparticles of BLT crystals prepared according to present teachings, panel A showing nanoparticles of undoped BLT and panel B showing nanoparticles of Fe-doped BLT. The scale bar in the pictures represent 100 nm.

Figure 8 is a line graph showing Particle Size Distribution of particles of Fe-doped BLT crystals (Fe:Ti 1:2 and Fe:Ti 0.25:2.75) in non-aqueous dispersions after milling according to present teachings, expressed as number percentage.

Figure 9 is a STEM image captured using a HR-SEM microscope of nanoparticles of Fe-doped BLT (Fe:Ti 1:2) crystals prepared according to present teachings, dispersed in a non-aqueous dispersion. The scale bar in the picture represents 20 nm.

Figure 10 is a STEM image captured using a HR-SEM microscope of nanoparticles of Fe-doped BLT (Fe:Ti 0.25:2.75) crystals prepared according to present teachings, dispersed in a non-aqueous dispersion. The scale bar in the picture represents 100 nm.

Figure 11 is a line graph showing Particle Size Distribution of swelled polymer matrix macroparticles containing nanoparticles of Fe-doped BLT (Fe:Ti 1:2 and Fe:Ti 0.25:2.75) prepared according to the present teachings, expressed as volume percentage.

Figure 12 is a STEM image captured using a HR-SEM microscope of swelled polymer matrix macroparticles including Fe-doped BLT (Fe:Ti 0.25:2.75) crystals prepared according to present teachings. The scale bar in the picture represents 200 nm.

Figure 13 is a line graph showing absorbance of non-aqueous dispersions comprising swelled polymer matrix macroparticles incorporating Fe-doped BLT (Fe:Ti 1:2 and Fe:Ti 0.25:2.75) nanoparticles according to the present teachings.

## DETAILED DESCRIPTION

The present disclosure, in at least some embodiments, provides compositions for protection against ultraviolet radiation, uses of such compositions and methods of making such compositions.

The UV protective compositions disclosed herein comprise Fe-doped BLT crystals having the formula $Bi_{(4-x)}La_{(x)}Ti_{(3-y)}Fe_{(y)}O_{12}$, wherein x is between 0.1 and 1.5; and wherein y is between 0 and 2, which when present as large particles (e.g., dimensions in each of the X-, Y- and Z-directions being greater than 200 nanometers (nm), resulting for instance in a hydrodynamic diameter of more than 200 nm as measured by DLS) may effectively absorb radiation having wavelengths of greater than about 400 nm. Accordingly, compositions comprising such large particles of composite BLT, whether or not further substituted by iron atoms, may provide protection against ultraviolet radiation having wavelengths up to at least 400 nm.

However, in the case in which the UV-protective composition is a sunscreen composition which comprises BLT, but which also contains particles that absorb light at wavelengths in the range of 400-800 nm, the sunscreen will be visible on the end-user because of the absorption in the visible range (> 400 nm).

It has surprisingly been found by the present Inventors that, although reduction of particle size of known inorganic UV-absorbing agents to dimensions of, for example below 1 micrometer ($\mu$m), typically below 100 nm (for instance, reduction to nanometric dimensions) is known to significantly reduce the maximum wavelength of light, including UV light, which is effectively absorbed by the particles, UV protective compositions according to the present teachings comprising particles of doped BLT crystals milled to nanoparticle size still provide substantial absorption of UV radiation of wavelength from 280 nm (or even shorter wavelength) up to about 400 nm, thus providing broad-spectrum protection against both UVA and UVB radiation, even in the absence of additional ultraviolet-absorbing agents.

Thus, in some embodiments, UV protective compositions disclosed herein, such as sunscreen compositions, comprise doped BLT crystals in the form of particles comprising one or more said crystals, wherein at least 90% of the particles are nanoparticles. In some embodiments, at least 95%, or at least 97.5% or at least 99% of the particles, in terms of number or volume of particles, are nanoparticles. In some embodiments, at least one dimension of the BLT crystal nanoparticles is expressed in terms of the hydrodynamic diameter as measured by DLS techniques.

In some embodiments, the cumulative particle size distribution in a sample is assessed in terms of the number of particles in the sample (denoted $D_N$). In some embodiments, the cumulative particle size distribution in a sample is assessed in terms of the volume of particles in the sample (denoted Dv).

In some embodiments, the maximum diameter of the nanoparticles is assessed for population distribution measured in terms of number of particles and percentage thereof. In some embodiments, the maximum diameter of the nanoparticles is assessed for population distribution measured in terms of sample volume of particles and percentage thereof.

In some embodiments, the doped BLT crystal nanoparticles are substantially invisible to the human eye, in particular when applied to the skin or hair of a subject, or if desired when applied to an inanimate surface, due to their small size.

In some embodiments, the doped BLT crystal nanoparticles are blended into a coloured composition and need not be substantially transparent and/or invisible, for instance when used in a make-up product, such as a foundation, which is slightly tinted when applied to the skin of a subject, or when used in a stain or paint applicable to inanimate surfaces.

According to some embodiments of the disclosure, there is provided a UV protective composition comprising Fe-doped BLT crystals, the level of doping by iron atoms being such that the Fe:Ti ratio can be between 1:50 and 1:2, in particular between 1:20 and 1:2.

According to a further aspect of some embodiments of the disclosure, there is provided a UV protective composition comprising doped BLT crystals for use in protecting the skin of a subject, such as a human subject, against ultraviolet radiation, in some embodiments providing broad-spectrum protection against both ultraviolet A and ultraviolet B radiation.

According to a further aspect of some embodiments of the disclosure, there is provided a UV protective composition comprising doped BLT crystals for use in protecting the hair of a subject, such as a human subject, against ultraviolet radiation, in some embodiments against both ultraviolet A and ultraviolet B radiation.

According to a further aspect of some embodiments of the disclosure, there is provided a method of protecting the skin of a subject against ultraviolet radiation, the method comprising applying to the skin of the subject an efficacious amount of a UV protective composition comprising doped BLT crystals. In some such embodiments, the UV-protective composition can be in the form of a skin-care product suitable for skin application and/or at least temporary retention thereupon.

According to a further aspect of some embodiments of the disclosure, there is provided a method of protecting the hair of a subject against ultraviolet radiation, the method comprising applying to the hair of the subject an efficacious amount of a UV protective composition comprising doped BLT crystals. In some such embodiments, the UV-protective composition can be in the form of a hair-care product suitable for hair application and/or at least temporary retention thereupon.

According to a further aspect of some embodiments of the disclosure, there is provided a method of protecting the surface of an inanimate object against ultraviolet radiation, the method comprising applying to the surface of the object an efficacious amount of a UV protective composition comprising doped BLT crystals. In some such embodiments, the UV-protective composition can be in the form of a coating product suitable for application to inanimate surfaces and/or at least temporary retention thereupon.

According to a further aspect of some embodiments of the disclosure, there is provided the use of doped BLT crystals in the manufacture of a composition for protection of the skin of a subject against ultraviolet radiation.

According to a further aspect of some embodiments of the disclosure, there is provided the use of doped BLT crystals in the manufacture of a composition for protection of the hair of a subject against ultraviolet radiation.

According to a further aspect of some embodiments of the disclosure, there is provided the use of doped BLT crystals in the manufacture of a composition for protection of surfaces of an object against ultraviolet radiation.

According to a further aspect of some embodiments of the disclosure, there is provided a method of manufacturing a UV protective composition, comprising combining doped BLT crystals, as an ultraviolet-absorbing agent, with other ingredients in proportions and in a manner suitable to make a UV-protective composition as described herein.

In some embodiments of the composition, use or method disclosed herein, the BLT crystals are present in the composition at a concentration of from about 0.001 wt.% to about 40 wt.%, from about 0.01 wt.% to about 30 wt.%, from about 0.1 wt.% to about 20 wt.% or from about 0.1 wt.% to about 15 wt.% of the final composition.

In some embodiments, the BLT crystals constitute at least 0.01 wt.%, at least 0.1 wt.%, at least 0.5 wt.%, at least 1 wt.%, at least 2 wt.%, at least 3 wt.%, at least 4 wt.%, at least 5 wt.%, at least 10 wt.%, at least 15 wt.%, at least 20 wt.%, at least 25 wt.%, at least 30 wt.%, or at least 35 wt.% of the composition. In some embodiments, the BLT crystals constitute at most 40 wt.%, at most 35 wt.%, at most 30 wt.%, at most 25 wt.%, at most 20 wt.%, at most 15 wt.%, at most 10 wt.%, at most 5 wt.%, at most 4 wt.%, at most 3 wt.%, at most 2 wt.%, at most 1 wt.%, at most 0.5 wt.%, or at most 0.1 wt.% of the composition.

In some embodiments of the composition, use or method disclosed herein, the doped BLT crystals are present in the composition as nanoparticles having at least one dimension of up to about 200 nm. In some embodiments, the nanoparticles have at least one dimension in the range of from about 10 nm to about 200 nm, from about 20 nm to about 150 nm, from about 20 to about 100 nm, from about 10 nm to about 80 nm, from about 10 to about 70 nm, from about 20 to about 70 nm, or from about 20 to about 60 nm, In some particular embodiments, the nanoparticles have at least one dimension of about 30 nm.

In some embodiments, the afore-mentioned dimensions or ranges of dimensions apply to at least 95%, or at least 97.5% or at least 99% of the population of the nanoparticles.

In some embodiments, the aforesaid smallest dimension of doped BLT crystals is estimated based on the hydrodynamic diameter of the particles as measured by DLS techniques. In some embodiments, the population distribution of the particles is expressed in terms of the cumulative particle size distribution, according to the number of particles in a sample. In some embodiments, the population distribution of the particles is expressed in terms of the cumulative particle size distribution of a sample volume of particles.

In some embodiments of the composition, use or method disclosed herein, the composition is generally devoid and/or generally free of an organic ultraviolet-absorbing agent.

In some embodiments of the composition, use or method disclosed herein, the composition is generally free of an organic ultraviolet-absorbing agent, that is to say the composition contains less than 5 wt.% organic UV-absorbing agents. In some embodiments the composition contains less than 4 wt.%, less than 3 wt.%, less than 2 wt.% or less than 1 wt.% organic UV-absorbing agents. In some embodiments the composition is largely free of organic ultraviolet-absorbing agents, *i.e.* the composition contains less than 0.5 wt.% organic UV-absorbing agents. In some embodiments the composition is mostly free of organic UV-absorbing agents, *i.e.* the composition contains less than 0.1 wt.% organic UV-absorbing agents. In some embodiments, the composition is principally free of organic ultraviolet-absorbing agents, *i.e.* the composition contains less than 0.05 wt.% organic UV-absorbing agents. In some embodiments, the composition is fundamentally free of organic UV-absorbing agents, *i.e.* the composition contains less than 0.01 wt.% organic UV absorbing agents. In some embodiments of the composition, use or method disclosed herein, the composition is generally devoid of organic ultraviolet-absorbing agents, considerably devoid of organic ultraviolet-absorbing agents, significantly devoid of organic ultraviolet-absorbing agents, substantially devoid of organic ultraviolet-absorbing agents, essentially devoid of organic ultraviolet-absorbing agents, substantively devoid of organic ultraviolet-absorbing agents or devoid of organic ultraviolet-absorbing agents.

In some embodiments of the composition, use or method disclosed herein, the composition is generally devoid and/or generally free of an additional inorganic ultraviolet-absorbing agent.

In some embodiments of the composition, use or method disclosed herein, the composition is generally free of an additional inorganic ultraviolet-absorbing agent, that is to say the composition contains less than 5 wt.% additional inorganic UV-absorbing agents. In some embodiments, the composition contains less than 4 wt.%, less than 3 wt.%, less than 2 wt.% or less than 1 wt.% additional inorganic ultraviolet-absorbing agents. In some embodiments, the composition is largely free of additional inorganic ultraviolet-absorbing agents, *i.e.* the composition contains less than 0.5 wt.% additional inorganic UV-absorbing agents. In some embodiments, the composition is mostly free of additional inorganic UV-absorbing agents, *i.e.* the composition contains less than 0.1 wt.% additional UV-absorbing agents. In some embodiments, the composition is principally free of additional inorganic ultraviolet-absorbing agents, *i.e.* the composition contains less than 0.05 wt.% additional UV-absorbing agents. In some embodiments, the composition is fundamentally free of additional inorganic UV-absorbing agents, *i.e.* the composition contains less than 0.01 wt.% additional UV absorbing agents.

In some embodiments of the composition, use or method disclosed herein, the composition is generally devoid of additional ultraviolet-absorbing agents, considerably devoid of additional ultraviolet-absorbing agents, significantly devoid of additional ultraviolet-absorbing agents, substantially devoid of additional ultraviolet-absorbing agents, essentially additional of organic ultraviolet-absorbing agents, substantively devoid of additional ultraviolet-absorbing agents or devoid of additional ultraviolet-absorbing agents.

In some embodiments of the composition, use or method disclosed herein, the doped BLT crystals are the sole ultraviolet-absorbing agent.

In some embodiments of the composition, use or method disclosed herein, the composition further comprises silver metal particles.

In some embodiments, the silver metal particles are present in the composition as nanoparticles. In some embodiments, the silver nanoparticles have at least one dimension of up to about 50 nm. In some embodiments, the silver nanoparticles have at least one dimension of up to about 40 nm. In some embodiments, the silver nanoparticles have at least one dimension of up to about 30 nm. In some embodiments, the silver nanoparticles have at least one dimension in the range of from about 10 nm to up to about 50 nm.

In some embodiments, the afore-mentioned dimensions or ranges of dimensions apply to at least 90%, or at least 95%, or at least 97.5% or at least 99% of the population of the silver nanoparticles.

In some embodiments, the aforesaid at least one dimension of the silver nanoparticles is estimated based on the hydrodynamic diameter of the particles as measured by DLS techniques. In some embodiments, the population distribution of the particles is expressed in terms of the cumulative particle size distribution according to the number of particles in a sample. In some embodiments, the population distribution of the particles is expressed in terms of the cumulative particle size distribution of a sample volume of particles.

In some embodiments, the silver nanoparticles are present in the composition at a concentration in the range of from about 0.01 wt.% to about 10 wt.% of the total composition. In some embodiments, the silver nanoparticles are present in the composition at a concentration in the range of from about 0.01 wt.% to about 5 wt.%, from about 0.05 wt.% to about 5 wt.%, or from about 0.1 wt.% to about 2 wt.% of the total composition. In some preferred embodiments, the silver nanoparticles are present in the composition at a concentration of about 1 wt.% or about 2 wt.% of the total composition.

In some embodiments, the silver particles constitute at least 0.01 wt.%, at least 0.1 wt.%, at least 0.5 wt.%, at least 1 wt.%, at least 2 wt.%, at least 3 wt.%, at least 4 wt.%, at least 5 wt.% or at least 10 wt.% of the composition. In some embodiments, the silver particles constitute at most 10 wt.%, at most 5 wt.%, at most 4 wt.%, at most 3 wt.%, at most 2 wt.%, at most 1 wt.%, at most 0.5 wt.%, or at most 0.1 wt.% of the composition.

In some embodiments of the composition, use or method disclosed herein, the UV protective composition is a composition for human or animal use, formulated as a topical composition. The topical composition may optionally be provided in a form selected from the group consisting of a cream, an emulsion, a gel, a lotion, a mousse, a paste and a spray. If desired, the topical composition can also be formulated into make-up cosmetics, for example, foundation, blusher, *etc.*

In some embodiments, the topical composition further comprises a dermatologically or cosmetically or pharmaceutically acceptable carrier.

In some embodiments, the topical composition further comprises one or more dermatologically or cosmetically or pharmaceutically acceptable additives or excipients, such as colorants, preservatives, fragrances, humectants, emollients, emulsifiers, waterproofing agents, surfactants, dispersants, thickeners, viscosity modifiers, anti-foaming agents, conditioning agents, antioxidants and the like. Such additives or excipients and the concentrations at which each can effectively accomplish its respective functions, are known to persons skilled in the pertinent art and need not be further detailed.

In some embodiments, the topical composition is a sunscreen composition.

In some embodiments, the UV protective composition is in the form of a coating that can be applied to the surface of an inanimate object. The coating composition may be provided in a form selected from the group consisting of liquid coat, an emulsion, a cream, a gel, a paste and a spray.

In another aspect of the present disclosure, there is provided a method for the preparation of the compositions disclosed herein.

According to a further aspect of some embodiments of the disclosure, there is provided a UV protective composition as disclosed herein, for use in protecting a subject, such as a human subject or a non-human animal, against a harmful effect of ultraviolet radiation, in some embodiments providing broad-spectrum protection against both ultraviolet A and ultraviolet B radiation.

In some embodiments, the composition is for use in protecting the skin of a subject, against a harmful effect of ultraviolet radiation, in some embodiments providing broad-spectrum protection against both ultraviolet A and ultraviolet B radiation.

In some embodiments, the composition is for use in protecting the hair of a subject, such as a human subject, against a harmful effect of ultraviolet radiation, in some embodiments against harmful effects of both ultraviolet A and ultraviolet B radiation.

The skin may be the skin of the face, of the arms, of the legs, of the neck of the torso, or of any other area of the body that can be exposed to UV radiation.

In some embodiments, the sunscreen composition as disclosed herein is applied to the skin of the subject prior to or during exposure to UV radiation. In some embodiments, the composition is reapplied intermittently, for example every 10 hours, every 9 hours, every 8 hours, every 7 hours, every 6 hours, every 5 hours, every 4 hours, every 3 hours, every 2 hours or every hour, or any intermediate value, during exposure to UV radiation.

In some embodiments, the UV-protective composition is for protecting the hair of a subject against ultraviolet radiation and is provided in a form selected from the group consisting of a cream, an emulsion, a gel, a lotion, a mousse, a paste and a spray. In some embodiments, the composition is provided in the form of a shampoo, a conditioner or a hair mask.

In some embodiments, the composition is formulated to be applied to the hair, or is applied to the hair, for a fixed period of time (such as up to 1 minute, up to 2 minutes, up to 3 minutes, up to 4 minutes or up to 5 minutes, up to 10 minutes, up to 15 minutes, up to 20 minutes, up to 25 minutes or up to 30 minutes) prior to rinsing. In some embodiments, the conditioner or hair mask is formulated for application to the hair, or is applied to the hair without rinsing, such that the conditioner or hair mask remains on the hair.

According to a further aspect of some embodiments of the disclosure, there is provided a UV protective composition as disclosed herein, for use in protecting an inanimate object, against a harmful effect of ultraviolet radiation, in some embodiments providing broad-spectrum protection against both ultraviolet A and ultraviolet B radiation.

According to a further aspect of some embodiments of the disclosure, there is provided a method of protecting the skin or the hair of a subject against a harmful effect of ultraviolet radiation, the method comprising applying to the skin and/or the hair of the subject a sunscreen composition comprising a matrix comprising a polymer and an oil; and particles of doped BLT crystals, dispersed in the matrix.

According to a further aspect of some embodiments of the disclosure, there is provided the use of a matrix comprising a polymer and an oil; and particles of a UV protective-agent comprising doped BLT crystals, dispersed in the matrix, in the manufacture of a composition for protection of the skin and/or the hair of a subject against a harmful effect of ultraviolet radiation.

According to a further aspect of some embodiments of the disclosure, there is provided the use of a matrix comprising a polymer and an oil; and particles of a UV protective-agent comprising doped BLT crystals, dispersed in the matrix, in the manufacture of a composition for protection of exterior surfaces of an inanimate object against a harmful effect of ultraviolet radiation. The exterior surface may comprise the surface of any porous or nonporous material, including, but not limited to glass, fabrics, leathers, woods, cardboards, metals, plastics, rubbers, ceramics and other structural materials.

The composition for the protection of inanimate objects against UV radiation, can be formulated in any form suitable for application to the surface of the inanimate object on which it is to be used.

EXAMPLES

Materials and Methods

**Materials**

The following materials were purchased from Sigma Aldrich, USA:

| | |
|---|---|
| $Bi_2O_3$ (99.9% pure) | CAS 1304-76-3 |
| $Fe_2O_3$ (99% pure) | CAS 1309-37-1 |
| $La_2O_3$ (99.9% pure) | CAS 1312-81-8 |
| Poly Acrylic Acid Sodium base (PAA) | CAS 9003-04-7 |

$TiO_2$ (99% pure)    CAS 13463-67-7

The milling media, namely Zirconia beads having an average diameter of 2mm, were purchased from Pingxiang Lier Ceramic Co., China.

**Equipment**

High Resolution Scanning Electron Microscope HSEM/TEM Magellan XHR 400L FE-SEM by Nanolab Technologies, Albany, New York, USA.

High Resolution X-ray diffractometer XRD Rigaku SmartLab® with Cu radiation generated at 40 kV and 30 mA (CuKa= 1.542 A) as the X-ray source.

Particle Size Analyzers (Dynamic Light Scattering) Zen 3600 Zetasizer (for particles in the range of up to about $10\mu m$) and Mastersizer 2000 (for particles in the range of $0.02\mu m$ to $2000\mu m$) by Malvern Instruments, Malvern, UK

Oven, Vulcan-Hart 3-1750 multi-stage programmable box furnace.

Temperature controllable circulating water bath, BL-30L 9 liter 1/3HP by MRC, Hampstead, London, UK

Grinding Mill Model HD-01 Attritor by Union Process®, Inc., Akron, Ohio, USA.

Analytical Balance XSE by Mettler-Toledo International Inc., Columbus, Ohio, USA.

Mortar Grinder Pulverisette 2 by Fritsch GmbH, Idar-Oberstein, Germany.

Double Planetary Mixer by Charles Ross & Son Company, Hauppauge, New York, USA.

Example 1: Preparation of BLT crystals

BLT crystals having the formula $Bi_{(4-x)}La_{(x)}Ti_{(3-y)}Fe_{(y)}O_{12}$ as an ultraviolet-absorbing agent, wherein x is between 0.1 and 1.5; and wherein y is between 0 and 2 were prepared by a solid solution method. The Fe-doped crystals included five different molar ratios of Fe to Ti, as follows: 0.0625:2.9375, 0.125:2.875, 0.25:2.75, 1:2 or 1.5:1.5.

In this process, the constituent metal oxides were mixed together in powder form so as to obtain the desired stoichiometric amount. $Bi_2O_3$, having a MW of 465.96 g/mol, $La_2O_3$ having a MW of 325.82 g/mol, $TiO_2$ having a MW of 79.87 g/mol were mixed in desired ratio so that the combined BLTO powders amounted to about 200 grams. When desired, $Fe_2O_3$ having a MW of 159.69 g/mol, was added while the amount of titanium dioxide was reduced, the amount of ferric oxide selected to provide the required doping ratio. The combination of metal oxides, which in case of intended iron doping can be termed the BLTO-Fe powders, amounted likewise to about 200 grams.

All materials were weighed using an analytical scale (Mettler Toledo, USA).

The powders of the constituent oxides were then mixed together for about 10 minutes at 70 rpm at ambient temperature in a Pulverisette 2 mortar grinder (Fritsch, Germany), so as to obtain homogeneously mixed powders (BLTO or BLTO-Fe, as appropriate). The mixed powders were transferred to a 500 ml alumina crucible and sintered or calcined by heating in a ceramic oven at a rate of 40°C per minute until the temperature reached 1000°C, and maintaining at this temperature for 24 hours, allowing for the formation of the desired doped or undoped BLT crystals. It is believed that under such conditions, the iron atoms can substitute the titanium atoms in the orthorhombic structure of the BLT to provide doping without breaking the crystallographic symmetry.

After 24 hours at 1000°C, the samples were allowed to cool down to ambient temperature (*circa* 23 °C), at which time they were again ground to homogeneous powder for about 10 minutes at 70 rpm by the Pulverisette 2 mortar grinder.

Powders of doped or undoped BLT crystals prepared as above-described were either used or analyzed "as is" in coarse form, or further size-reduced and used and analyzed in the form of nanoparticles, as described in following examples. It is to be understood that the coarse material was manually ground with a mortar and pestle to disassociate any gross agglomerate that may be present in the resulting powders, so as to eliminate coarse lumps of particles. In bulk size, the BLT compounds displayed a pale yellow shade if undoped and a reddish tint if doped, the color intensity depending on the degree of iron doping.

Example 2: Crystal Structure Determination

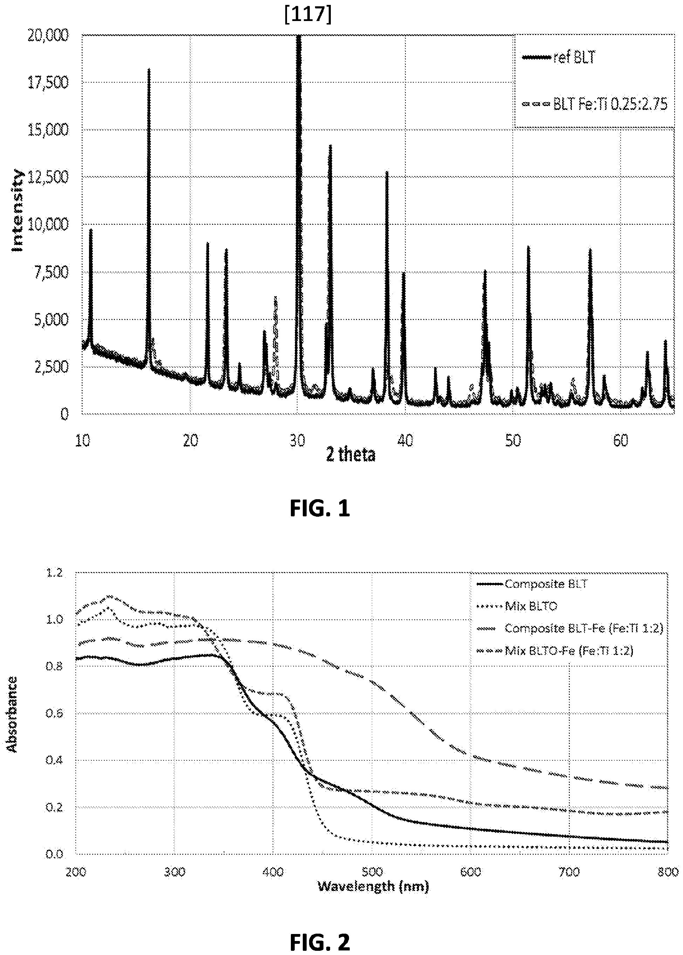

The crystal structure of doped BLT crystals for a Fe:Ti substitution of 0.25:2.75 as above-prepared was determined by powder XRD using Rigaku TTRAX-III X-ray diffractometer. The X-ray source (Cu anode) was operated at a voltage of 40 kV and a current of 30 mA on packed powder samples. Data were collected in continuous detector scan mode at a step size of 0.02°/step. Diffractograms were collected over the 2Θ range of 10° to 65°. The results are shown in **Figure 1,** wherein the pattern of undoped BLT crystals is displayed as a continuous line, whereas that of Fe:Ti 0.25:2.75 doped BLT crystals is shown as a dotted line. For both materials, a predominant peak is seen around 2Θ of about 30° and doping did not significantly affect the crystalline peaks characteristic of the BLT crystals.

Example 3: Absorbance Determination in Powder

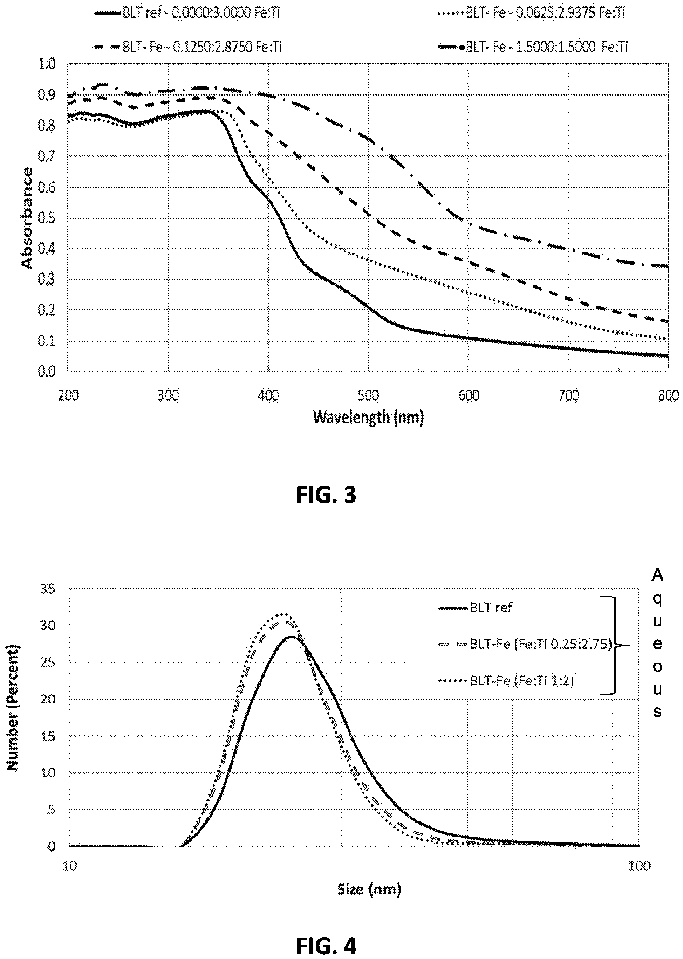

Absorbance correlation of coarse powders over the wavelength range of 200--800 nm was calculated using a Cary 300 UV-Vis spectrophotometer with an integrated sphere detector (Agilent Technologies, Santa Clara, CA, USA).
Briefly, the absorbance of the samples was qualitatively estimated by subtracting the amount of light reflected from the powder sample, gathered by the integrated sphere detector of the spectrophotometer, from the amount of light reflected from a white surface (which reflects all incident light). Since the extent of penetration of the light into the samples and the extent of scattering of the sample is unknown, this measurement provides an absorbance profile of the sample rather than a true quantitative measurement.
Results, showing correlation to absorbance as a function of wavelength, determined by diffuse reflection measurement gathered by the integrated sphere method, are presented in Figures 2 and 3.
**Figure 2** shows the absorbance of doped (Fe:Ti 1:2) or undoped BLT crystals, as obtained following the sintering method of Example 1, as compared to their respective mixture of constituent metal oxides. As seen in the figure, the sintered materials differ from the initial mix of the constituents. Whereas the constituent mixtures display "step-like" variations in absorbance, each step predominantly attributable to one or another of the individual constituents, the formed crystals display much smoother variation curves. Undoped BLT crystals show a relatively constant UV absorbance of about 0.84 from 200 nm to about 350 nm, with a relatively even decrease till about 550 nm and with a still relatively high absorbance of about 0.56 at 400 nm, this absorbance representing about 67% of the initial plateau value of about 0.84. Doped (Fe:Ti 1:2) BLT crystals, show a relatively constant UV absorbance of about 0.90 from 200 nm to about 415 nm, suggesting that the Fe-doped BLT may provide for a broader range of UV protection.
**Figure 3** shows the impact of varying degrees of doping on the absorbance of BLT crystals, all such coarse powders having been prepared according to Example 1. As seen in the figure, where only part of the doped BLT crystal samples are shown for clarity, the higher the degree of doping in the range tested, the higher the initial "plateau" absorbance and/or the broader the UV range over which such materials significantly absorb radiation. Whereas undoped BLT shows a relatively constant UV absorbance of about 0.84 from 200 nm to about 350 nm, its Fe:Ti 0.0625:2.9375 doped variant displays an approximate average absorbance of 0.82 from 200 nm to about 380 nm, whereas the Fe:Ti 0.125:2.875 doped variant displays an average absorbance of about 0.88 from 200 nm to about 380 nm and the Fe:Ti 1.5:1.5 doped variant displays an average absorbance of about 0.91 from 200 nm to about 430 nm.

Example 4: Preparation of nanoparticles

Nanoparticles of doped or undoped BLT crystals, as well as their respective constituents and mixtures thereof when desired, were prepared from the ground samples obtained in Example 1 or from their stock powders. Generally, all such samples or stock powders contained particles having a size greater than about 5 micrometer ($\mu$m) and may be referred hereinafter as the coarse materials. The coarse powders were milled in an Attritor grinding mill (HD-01 by Union Process®) using a batch size of 200 g with solid loading 10% (20 g) as follows.
All materials were weighed using an analytical scale (XSE by Mettler Toledo). 20 g of PAA dispersant was weighed and dispersed in about 100 ml of deionized water. 20 g of coarse powder was weighed and introduced into the dispersant-containing liquid to provide a dispersant to inorganic material ratio of 1:1 yielding a slurry of the inorganic material. Water was added to complete batch size to 200 g, the solids constituting about 10 wt.% of the sample.
The aqueous slurry of inorganic material was then placed in a zirconia pot with 2300 g of 2 mm diameter zirconia grinding beads. The pot was placed in the grinding mill, and the grinding mill activated at 700 rpm for about 75 hours at 25°C.

The hydrodynamic diameter of the nanoparticles obtained by this method was determined by Dynamic Light Scattering, using a Zen 3600 Zetasizer from Malvern Instruments Ltd. (Malvern, UK). A sample of the milled nanoparticles was further diluted in deionized water to form a suspension having a solid concentration of about 0.5 wt.%.

Representative results, showing the percentage of number of doped and undoped BLT crystal particles having hydrodynamic diameters in the range of 1-100 nm are presented in Figure 4.

As shown in the figure, the particles of inorganic material in suspension had hydrodynamic diameters of up to about 100 nm. The majority of doped and undoped BLT crystal particles had hydrodynamic diameters in the size range of from about 15 nm and up to about 60 nm or 50 nm. The predominant peak of undoped BLT, was around about 26 nm, whereas the two Fe-doped variants displayed similar peaks at about 28 nm for Fe:Ti 0.25:2.75 and about 24 nm for Fe:Ti 1:2 Results of the particle size distribution of the nanoparticles prepared as herein described, namely the maximum hydrodynamic diameter of a percentage of the population, are provided in the Table 1 below, in terms of percent of number of particles.

**Table 1**

| Material | Max. Hydrodynamic Diameter (nm) | | | | |
|---|---|---|---|---|---|
| | 50.0% | 90.0% | 95.0% | 97.5% | 99.0% |
| Undoped BLT Fe:Ti 0:3 | 25.6 | 37.0 | 43.1 | 53.0 | 72.0 |
| Doped BLT Fe:Ti 0.25:2.75 | 28.0 | 34.0 | 37.7 | 44.9 | 64.6 |
| Doped BLT Fe:Ti 1:2 | 24.0 | 33.0 | 36.5 | 43.0 | 68.3 |

As can be seen from the above table, at least 99% of the nanoparticles of doped or undoped BLT as prepared and size-reduced according to the present teachings have a dimension of at most 100 nm.

Example 5: Absorbance of suspended crystal nanoparticles

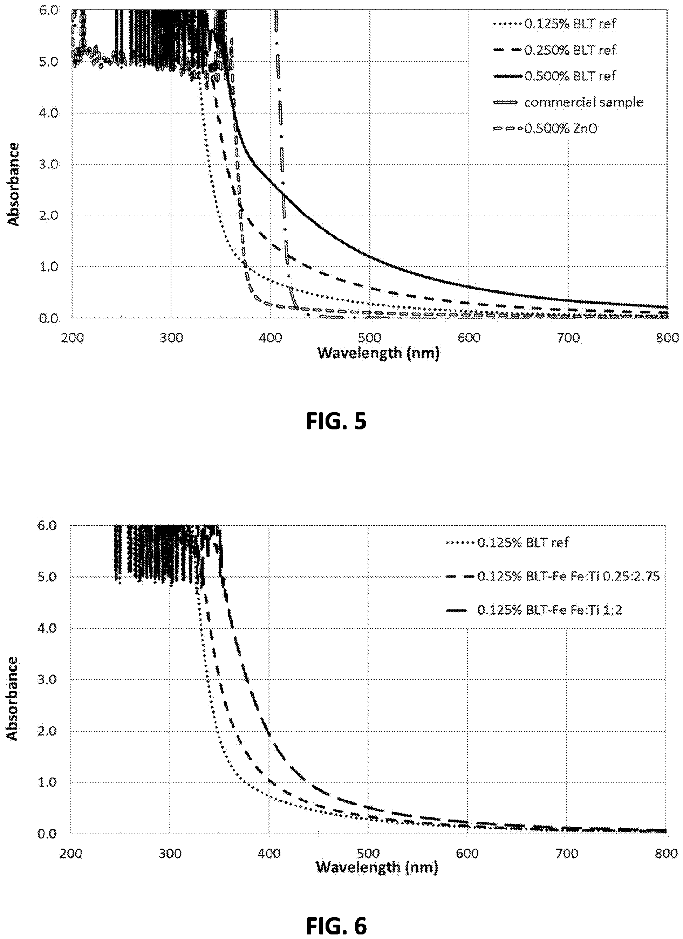

Absorbance of the doped and undoped BLT crystal nanoparticles prepared according to Example 4 was measured over the wavelength range of 200-800 nm using a Cary 300 UV-Vis spectrophotometer with quartz cuvette (10 mm light pathway). The samples were diluted in the vehicle in which the inorganic materials were milled (namely with deionized water containing 20 wt.% PAA) to provide any desired predetermined solid concentration (e.g., 0.125 wt.%, 0.25 wt.% and 0.5 wt.%,). Results are presented in Figures 5 and 6. For convenience, it should be recalled that an absorbance value of 1 indicates a UV blocking of at least about 90%, whereas an absorbance value of 2 indicates blocking of up to 99% of the radiation.

In **Figure 5,** the absorbance in the 280-400 nm wavelength range is shown for undoped BLT nanoparticles at increasing concentrations as compared to a commercial sample (Skingard® sunscreen composition of Careline®) and to a nanoparticulated control consisting of 0.5 wt.% ZnO prepared by a similar method and having a $D_N50$ of about 25 nm. As can be seen in the figure, the control zinc oxide and commercial sample displayed a steeper drop in absorbance than the present composite material. Undoped BLT crystals displayed a very significant absorbance up to at least 400 nm at all concentrations tested. While at 400 nm the absorbance provided by 0.5 wt.% of ZnO was of only about 0.27, undoped BLT displayed at this same wavelength an absorbance of about 0.74, 1.47 and 2.66 for compositions containing solid concentrations of 0.125 wt.%, 0.25 wt.% and 0.5 wt.%, respectively. Thus, at the same 0.5 wt.% concentration as the zinc oxide control, the BLT crystals prepared according to the present teachings displayed a ten-fold higher value, which indicates a much more significant difference in absorbance efficiency. Moreover, it can be seen that increasing the concentration of BLT in the tested range, resulted in a broadening of the ranges of wavelengths wherein the composite provided radiation absorbance.

Since 0.125 wt.% of undoped BLT already provides for a significant absorbance of about 0.74 at 400 nm, the absorbance of Fe-doped BLT crystals (Fe:Ti 0.25:2.75 and Fe:Ti 1:2), is displayed in **Figure 6** only at this concentration. As can be seen in the figure, a higher level of substitution of titanium atoms being replaced by iron atoms led to absorbance over a broader spectrum and/or a higher absorbance at any particular wavelength within the range of efficiency. For instance, while 0.125 wt.% of undoped BLT provided for an absorbance of about 0.74 at 400 nm, the same concentration of Fe-doped BLT (Fe:Ti 0.25:2.75 and Fe:Ti 1:2) respectively displayed absorbance of 1.06 and 1.95.

Higher concentrations of nanoparticles of Fe-doped BLT were also tested and displayed a pattern similar to that of unsubstituted BLT, namely over the range tested a higher concentration of material led to a broader range of wavelength with efficient absorbance.

Example 6: Scanning electron microscope studies

The doped and undoped BLT crystal nanoparticles were also studied by High Resolution Scanning Electron Microscopy (HR-SEM) using Magellan™ 400 HSEM/TEM by Nanolab Technologies.

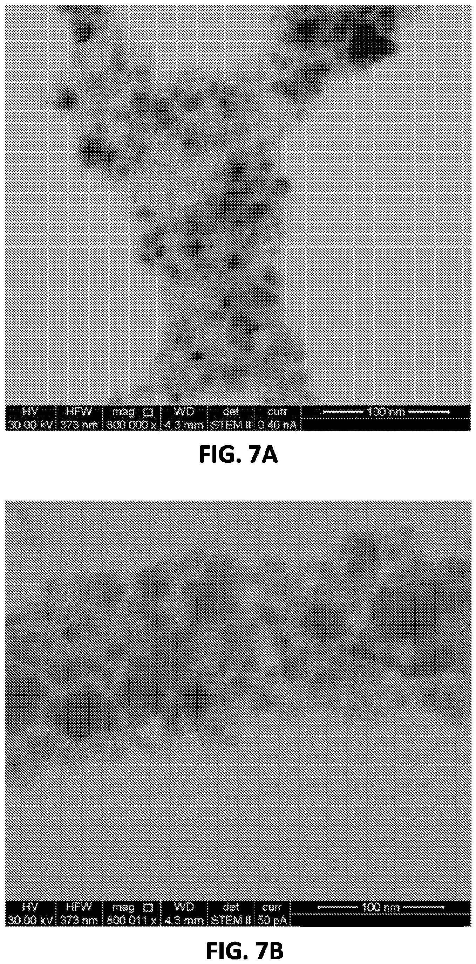

**Figure 7A** shows an image for undoped BLT crystal nanoparticles, wherein **Figure 7B** shows an image for Fe-doped BLT crystal nanoparticles (Fe:Ti 1:2).

Example 7: Determination of critical wavelength

Based on the absorbance spectra determined according to previous Examples, critical wavelength was calculated for undoped BLT crystals and for two Fe-doped variants, all measured at nanoparticle concentration of 0.5 wt.% and 0.125 wt.%. A suspension of nanoparticles of Zinc Oxide at 0.5 wt.% served as control.

Briefly, in order to quantify the breadth of UV protection, the absorbance of the sunscreen composition was integrated from 290 nm to 400 nm the sum reached defining 100% of the total absorbance of the sunscreen in the UV region. The wavelength at which the summed absorbance reaches 90% absorbance was determined as the 'critical wavelength' which provided a measure of the breadth of sunscreen protection.

The critical wavelength $\lambda_c$ was defined according to the following equation:

$$\int_{290}^{\lambda_c} \mathrm{lg}[1/T(\lambda)]\,d\lambda = 0.9 \cdot \int_{290}^{400} \mathrm{lg}[1/T(\lambda)]\,d\lambda$$

wherein:

$\lambda_c$ is the critical wavelength;
$T(\lambda)$ is the mean transmittance for each wavelength; and
$D\lambda$ is the wavelength interval between measurements.

Critical wavelengths as calculated are presented in Table 2 below.

**Table 2**

| | Critical Wavelength (nm) | |
|---|---|---|
| **Inorganic Material** | 0.125 wt.% | 0.5 wt.% |
| BLT undoped | 370 | 390 |
| BLT-Fe Fe:Ti 0.25:2.75 | 374 | 393 |
| BLT-Fe Fe:Ti 1:2 | 378 | 397 |
| ZnO Control | Not Available | 362 |

As can be seen from the above table, according to the Critical Wavelength Method, undoped and Fe-doped BLT crystal nanoparticles can be classified as providing broad spectrum protection (*i.e.* having a critical wavelength of 370 nm or more) at concentrations of as low as 0.125 wt.% and 0.5 wt.% Such results are superior to those achieved by the control suspension consisting of ZnO nanoparticles having similar particle size distribution when tested at the higher concentration of 0.5 wt.%.

Example 8: Preparation of composition comprising polymer matrix and BLT crystals

The nanoparticles of doped or undoped BLT crystals prepared according to the present teachings and above-examples can be further processed so as to be embedded or immobilized within a polymer matrix. Suitable methods and polymers are described by the present Applicant in PCT Publication No. WO 2017/013633. In particular, Example 2 of the reference provides for the preparation of a polymer matrix, whereas Example 3 teaches how to blend such matrix with nanoparticles, and how to further process such mixture so as to obtain polymer embedded particles.

Example 9: Preparation of composition comprising BLT crystals in wood lacquer

Doped and undoped BLT crystal nanoparticles are diluted in a clear wood lacquer (Tambour Clear Glossy Lacquer for Wood No. 8, Cat. No. 149-001) to a particle concentration of 1% by weight of the total lacquer composition. The resulting mixtures are sonicated for 30 seconds using a Misonix Sonicator tip (Misonix, Inc.) at amplitude 100, 15 W. The sonicated lacquer dispersions are applied upon a microscopic glass slide at an initial thickness of about 100 $\mu$m (using 100 $\mu$m thick spacers and a leveling rod). The lacquer coated slides are left to dry for at least 12 hours at ambient temperature (circa 23°C) resulting in a dried layer of sample of about 5 $\mu$m. The lacquer devoid of added nanoparticles serves as control. Absorbance of the dried layers of lacquer over the wavelength range of 200-800 nm is assessed using a Cary 300 UV-Vis spectrophotometer.

Example 10: Non-aqueous compositions comprising doped BLT crystals

The Fe doped BLT crystals were prepared as described in Examples 1 to 3.

**Preparation of nanoparticles**

Nanoparticles of doped BLT crystals were prepared from the ground samples obtained in Example 1. Generally, all such samples contained particles having a size greater than about 5 micrometer ($\mu$m) and may be referred hereinafter as the coarse materials. The coarse powders were milled in an Attritor grinding mill (HD-01 by Union Process®) using a batch size of 300 g with solid loading 10% (30 g) as follows.

All materials were weighed using an analytical scale (XSE by Mettler Toledo). 30 g of polyhydroxystearic acid (commercially available from Innospec Performance Chemicals as Dispersun DSP-OL100 or Dispersun DSP-OL300) dispersant was weighed and dispersed in about 100 ml of Isopar™ L of ExxonMobil Chemicals or C12-C15 alkyl benzoate (commercially available from Phoenix Chemical as Pelemol® 256). 30 g of coarse powder of Fe-doped BLT was weighed and introduced into the dispersant-containing liquid to provide a dispersant to inorganic material weight per weight ratio of 1:1 yielding a slurry of the inorganic material. Isopar™ L or C12-C15 alkyl benzoate was added to complete batch size to 300 g, the solids constituting about 10 wt.% of the sample.

The oily slurry of inorganic material was then placed in a zirconia pot with 2300 g of 2mm diameter zirconia grinding beads. The pot was placed in the grinding mill, and the grinding mill activated at 700 rpm for about 75 hours at 25°C.

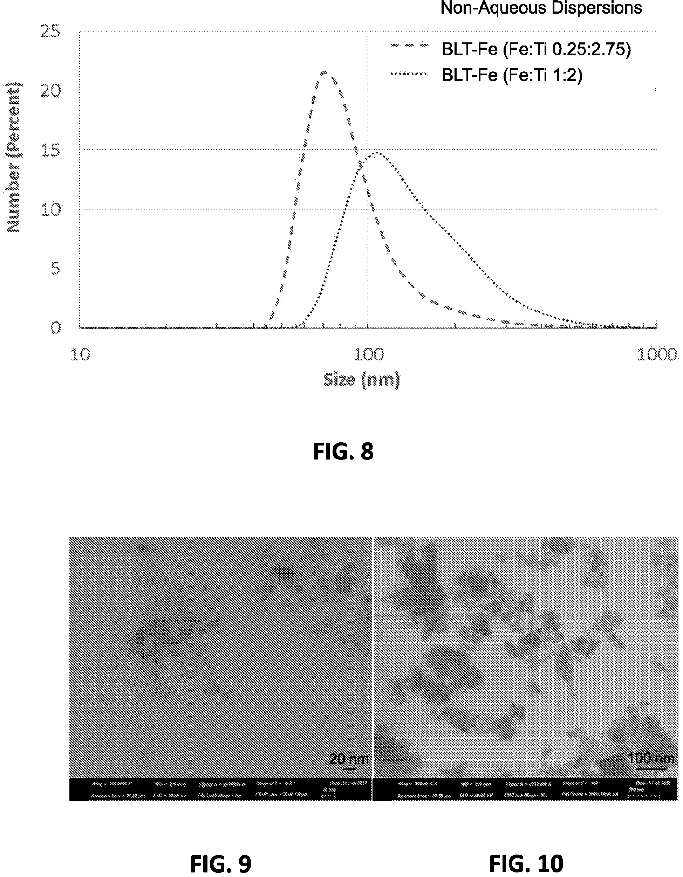

The hydrodynamic diameter of the milled particles was determined by Dynamic Light Scattering, using a Malvern Nano ZS Zetasizer particle size analyzer. A sample of the milled nanoparticles was further diluted in Isopar® L to form a suspension having a solid inorganic concentration of about 0.1 wt.% for the sake of such measurements. Representative results, showing the hydrodynamic diameters of Fe-doped BLT particles, having Fe:Ti doping of 1:2 or 0.25:2.75, expressed in terms of percentage of number of particles in the range of 10-1,000 nm are presented in **Figure 8.** The sample including the BLT doped at Fe:Ti of 1:2 is represented by the dispersion prepared using DSP-OL300 in Isopar® L, while the sample including the BLT doped at Fe:Ti of 0.25:2.75 is represented by the dispersion prepared using DSP-OL100 in C12-C15 alkyl benzoate. Other dispersions using the alternative combinations of dispersants and non-aqueous / oily carriers gave similar distributions. No peaks were observed outside the presented range.

As shown in **Figure 8,** the milled particles of solid inorganic crystals in non-aqueous suspensions had hydrodynamic diameters of up to about 500 nm. The majority of BLT nanoparticles Fe:Ti doped at 0.25:2.75 had hydrodynamic diameters in the size range of from about 40 nm and up to about 300 nm, with a predominant peak around about 70 nm. The majority of BLT particles Fe:Ti doped at 1:2 had hydrodynamic diameters in the size range of from about 60 nm and up to about 500 nm, with a predominant peak around about 110 nm. Results of the particle size distribution of the nanoparticles prepared as herein described, namely the maximum hydrodynamic diameter of a percentage of the population, are provided in the Table 3 below, in terms of percent of number of particles.

**Table 3**

| Material | Max. Hydrodynamic Diameter | | |
|---|---|---|---|
| | $D_N10$ | $D_N50$ | $D_N90$ |
| BLT 1:10 | 58.0 nm | 79.5 nm | 138.0 nm |
| BLT 1:2 | 84.3 nm | 128.0 nm | 256.0 nm |

As the above dynamic light scattering measurements, which assume, for the sake of hydrodynamic diameter calculations, that the particles are perfect spheres tend to overestimate the actual size of the particles, in particular if non-spherical, the size of the particles of Fe-doped BLT was further assessed by STEM microscopy. **Figure 9** and **Figure 10** are STEM images of particles of Fe-doped BLT, having a Fe:Ti doping ratio of 1:2 and 0.25:2.75, respectively. It can be seen from the images that the real size of the nanoparticles is below 100 nm for both types of Fe-doped BLT nanoparticles in the non-aqueous dispersions.

Example 11: Preparation of composition comprising swelled polymer matrix macroparticles and nanoparticles UV-protective agent

2 weight portions of a swelled polymer matrix (consisting of Nucrel® 699 and Isopar™ L), prepared as described in Example 3 of WO 2017/013633 to the same Applicant, were mixed with 1 weight portion of non-aqueous dispersions containing 10 wt.% inorganic nanoparticles of UV-protective agents, Fe-doped BLT having a Fe:Ti ratio of 1:2 or 0.25:2.75, prepared as described in Example 10. The oil dispersions used herein are those which served for the measurements illustrated in Figure 8. 60-80 g Isopar™ L were added to the mixture of swelled polymer matrix and oil dispersed inorganic nanoparticles of Fe-doped BLT to give a final weight of 200 g.

200 g of the resulting mixture were placed in a zirconia pot, 2,500 g of zirconia beads of about 2.38 mm (3/32") diameter were added to the pot, and the pot was placed in the grinding mill. The temperature of the pot was maintained at 25°C while the grinding mill was set to mill the contents of the pot at 700 rpm for 12 hours resulting in a composition according to the teachings herein comprising inorganic nanoparticles of UV-protective agent dispersed and embedded in the swelled polymer matrix macroparticles.

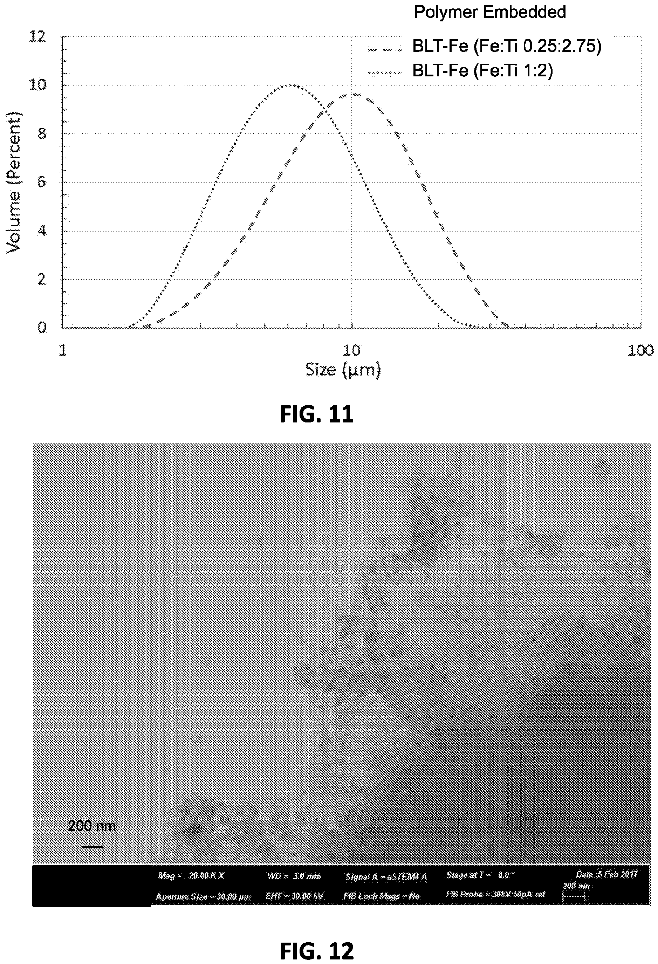

The hydrodynamic diameters of the resulting macroparticles of swelled polymer matrix were determined using Malvern Mastersizer 2000. The percentage (per volume) of macroparticles of polymer embedding the BLT-Fe doped nanoparticles are presented in **Figure 11** in the range of 1-100 $\mu$m. No peaks were observed outside of the presented range. STEM microscopic analysis performed using HR-SEM confirmed that the UV-protective nanoparticles of Fe-doped BLT were incorporated inside the polymeric macroparticles, as can be seen in **Figure 12** illustrating the embedment of BLT nanoparticles Fe-doped at a Fe:Ti ratio of 0.25:2.75.

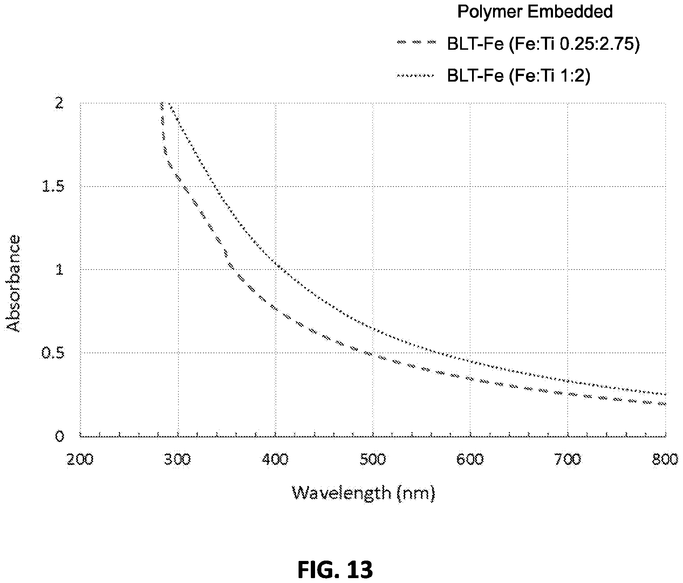

The absorbance of the non-aqueous dispersions of polymer embedded Fe-doped BLT composite lotions was measured as described in Example 3 with the following modifications. The dispersions were spread between two quartz slides (76.2x25.4x1.0mm) and their absorbance over the wavelength range of 200-800 nm was assessed using a Cary 300 UV-Vis spectrophotometer. The results are presented in **Figure 13.** Both dispersions containing at most about 2 wt.% of Fe-doped BLT significantly absorbed UV in the range of up to 400nm.

**Claims**

1. A UV-protective composition comprising one or more Lanthanum-Modified Bismuth Titanate (BLT) crystals each independently having the chemical formula $Bi_{(4-x)}La_{(x)}Ti_{(3-y)}Fe_{(y)}O_{12}$ as an ultraviolet-absorbing agent, wherein x is between 0.1 and 1.5; and wherein y is between >0 and 2, wherein the BLT crystals are in the form of nanoparticles consisting of one or more said crystals, at least 50% of the total number of said nanoparticles having at least one dimension of up to 200 nm.

2. The composition according to any one of claim 1 wherein x is between 0.5 and 1.0, suitably wherein x is between 0.7 and 0.8.

3. The composition according to any one of claim 1 or claim 2, wherein y is between 0.01 and 1.8.

4. The composition according to any one of claim 1 to claim 3, wherein a molar ratio of Fe to Ti is selected from (0.0625 and 2.9375), (0.125 and 2.875), (0.25 and 2.75), (1 and 2) and (1.5 and 1.5).

5. The composition according to any one of claim 1 to claim 4, wherein at least 90%, of the total number of said nanoparticles having at least one dimension of up to about 200 nm, up to about 150 nm, or up to about 100 nm, said nanoparticles optionally consisting of crystals having the same chemical formula.

6. The composition according to any one of claim 1 to claim 5, wherein the one or more BLT crystals are present in the composition in the form of nanoparticles at a concentration in the range of from about 0.001 wt.% to about 40 wt.% of the total composition.

7. The composition according to any one of claim 1 to claim 6, wherein said nanoparticles of said one or more BLT crystals are dispersed with a dispersant in a polymer matrix further comprising a thermoplastic polymer swelled with an oil.

8. The composition according to claim 7, said polymer matrix is in the form of polymer matrix flakes wherein each flake of said polymer matrix flakes has a flake length (Lf), a flake width (Wf), and a flake thickness (Tf), said polymer matrix flakes having a dimensionless flake aspect ratio (Rf) defined by: Rf = (Lf•Wf)/(Tf)$^2$, wherein, with respect to a representative group of at least ten polymer matrix flakes, an average Rf is at least 5; and wherein the nanoparticles within said representative group have an average particle size (D50) of at most 100 nm.

9. The composition according to any one of claim 7 or claim 8, wherein the dispersant adapted to disperse the nanoparticles of BLT crystals within said polymer matrix has a hydrophilic-lipophilic balance (HLB) value of at most 9, generally at most 6, suitably at most 4, and more suitably at most 3.

10. The composition according to any one of claim 7 to claim 9, wherein the thermoplastic polymer in the polymer matrix comprises at least one ethylene-acrylic (EAA) polymer, ethylene-methacrylic (EMMA) polymer, ethyl vinyl acetate (EVA) polymer, or combinations thereof.

11. The composition according to any one of claim 1 to claim 10, wherein the absorption spectrum of the composition has a critical wavelength of at least 370 nm.

12. The composition according to any one of claim 1 to claim 11, wherein the area under the curve (AUC) formed by the UV-absorption of the one or more BLT crystals as a function of wavelength in the range of 280 nm to 400 nm ($AUC_{280-400}$) is at least 75%, at least 85% or at least 95% of the AUC formed by the same crystals at the same concentration in the range of 280 nm to 700 nm ($AUC_{280-700}$).

13. The composition according to any one of claim 1 to claim 12, formulated as one of the following: (a) a skin-care composition for application to human or non-human animal skin; (b) a hair-care composition for application to human or non-human animal hair; or (c) a coating composition for application to an inanimate surface.

14. Use of the composition according to any one of claim 1 to claim 13, for protecting the hair of a subject or for protecting an inanimate object against a harmful effect of ultraviolet radiation, in particular protecting against ultraviolet A radiation and ultraviolet B radiation.

15. The composition according to any one of claim 1 to claim 13 for use in protecting the skin of a subject against a harmful effect of ultraviolet radiation, in particular protecting against ultraviolet A radiation and ultraviolet B radiation.


**Patentansprüche**

1. UV-Schutzzusammensetzung, umfassend ein oder mehrere Lanthan-modifizierte Bismut-Titanat-Kristalle (BLT-Kristalle), die jeweils unabhängig die chemische Formel $Bi_{(4-x)}La_{(x)}Ti_{(3-y)}Fe_{(y)}O_{12}$ als ultraviolettabsorbierendes Mittel aufweisen, wobei x zwischen 0,1 und 1,5 ist; und wobei y zwischen >0 und 2 ist, wobei die BLT-Kristalle in Form von Nanopartikeln sind, die aus einem oder mehreren dieser Kristalle bestehen, wobei mindestens 50 % der Gesamtanzahl dieser Nanopartikel mindestens eine Dimension von bis zu 200 nm aufweisen.

2. Zusammensetzung nach einem der Ansprüche 1, wobei x zwischen 0,5 und 1,0 ist, auf geeignete Weise wobei x zwischen 0,7 und 0,8 ist.

3. Zusammensetzung nach einem der Ansprüche 1 oder 2, wobei y zwischen 0,01 und 1,8 ist.

4. Zusammensetzung nach einem der Ansprüche 1 bis 3, wobei das Molverhältnis von Fe zu Ti ausgewählt ist aus (0,0625 und 2,9375), (0,125 und 2,875), (0,25 und 2,75), (1 und 2) und (1,5 und 1,5).

5. Zusammensetzung nach einem der Ansprüche 1 bis 4, wobei mindestens 90 % der Gesamtanzahl der Nanopartikel mindestens eine Dimension von bis zu etwa 200 nm, bis zu etwa 150 nm oder bis zu etwa 100 nm aufweisen, wobei die Nanopartikel optional aus Kristallen bestehen, die die gleiche chemische Formel aufweisen.

6. Zusammensetzung nach einem der Ansprüche 1 bis 5, wobei das eine oder die mehreren BLT-Kristalle in der Zusammensetzung in Form von Nanopartikeln in einer Konzentration in dem Bereich von etwa 0,001 Gewichtsprozent bis etwa 40 Gewichtsprozent der Gesamtzusammensetzung vorhanden sind.

7. Zusammensetzung nach einem der Ansprüche 1 bis 6, wobei die Nanopartikel des einen oder der mehreren BLT-Kristalle mit einem Dispergiermittel in einer Polymermatrix dispergiert sind, ferner umfassend ein thermoplastisches Polymer, das mit einem Öl gequollen ist.

8. Zusammensetzung nach Anspruch 7, wobei die Polymermatrix in Form von Polymermatrixflocken ist, wobei jede Flocke der Polymermatrixflocken eine Flockenlänge (Lf), eine Flockenbreite (Wf) und eine Flockenstärke (Tf) aufweist, wobei die Polymermatrixflocken ein dimensionsloses Flocken-Seitenverhältnis (Rf) aufweisen, das definiert ist durch: $Rf = (Lf \cdot Wf)/(Tf)^2$, wobei, bezogen auf eine repräsentative Gruppe von mindestens zehn Polymermatrixflocken, ein durchschnittliches Rf mindestens 5 ist; und wobei die Nanopartikel innerhalb der repräsentativen Gruppe eine durchschnittliche Teilchengröße (D50) von höchstens 100 nm aufweisen.

9. Zusammensetzung nach einem der Ansprüche 7 oder 8, wobei das Dispergiermittel, das zum Dispergieren der Nanopartikel aus BLT-Kristallen in der Polymermatrix angepasst ist, einen Hydrophil-Lipophil-Gleichgewichtswert (HLB-Wert) von höchstens 9, generell höchstens 6, auf geeignete Weise höchstens 4 und auf noch geeignetere Weise von höchstens 3 aufweist.

10. Zusammensetzung nach einem der Ansprüche 7 bis 9, wobei das thermoplastische Polymer in der Polymermatrix mindestens ein Ethylen-Acryl-Polymer (EAA-Polymer), Ethylen-Methacryl-Polymer (EMMA-Polymer), Ethyl-Vinylacetat-Polymer (EVA-Polymer) umfasst, oder Kombinationen davon.

11. Zusammensetzung nach einem der Ansprüche 1 bis 10, wobei das Absorptionsspektrum der Zusammensetzung eine kritische Wellenlänge von mindestens 370 nm aufweist.

12. Zusammensetzung nach einem der Ansprüche 1 bis 11, wobei die Fläche unter der Kurve (AUC), die durch die UV-Absorption des einen oder der mehreren BLT-Kristalle abhängig von der Wellenlänge in dem Bereich von 280 nm bis 400 nm ($AUC_{280-400}$) gebildet wird, mindestens 75 %, mindestens 85 % oder mindestens 95 % der AUC ist, die von den gleichen Kristallen bei der gleichen Konzentration in dem Bereich von 280 nm bis 700 nm ($AUC_{280-700}$) gebildet wird.

13. Zusammensetzung nach einem der Ansprüche 1 bis 12, formuliert als eine der Folgenden: (a) eine Hautpflegezusammensetzung zur Anwendung auf menschliche oder nicht menschliche tierische Haut; (b) eine Haarpflegezusammensetzung zur Anwendung auf menschliches oder nicht menschliches tierisches Haar; oder (c) eine Beschichtungszusammensetzung zur Anwendung auf eine unbelebte Oberfläche.

14. Verwendung der Zusammensetzung nach einem der Ansprüche 1 bis 13 zum Schutz des Haars eines Subjekts oder eines unbelebten Objekts gegen die schädliche Wirkung von Ultraviolettstrahlung, insbesondere zum Schutz gegen Ultraviolett-A-Strahlung und Ultraviolett-B-Strahlung.

15. Zusammensetzung nach einem der Ansprüche 1 bis 13 zur Verwendung zum Schutz der Haut eines Subjekts gegen eine schädliche Wirkung von Ultraviolettstrahlung, insbesondere zum Schutz gegen Ultraviolett-A-Strahlung und Ultraviolett-B-Strahlen.

**Revendications**

1. Composition de protection contre les UV comprenant un ou plusieurs cristaux de titanate de bismuth modifié au lanthane (BLT) ayant chacun indépendamment la formule chimique $Bi_{(4-x)}La_{(x)}Ti_{(3-y)}Fe_{(y)}O_{12}$ en tant qu'agent d'absorption des ultraviolets, dans laquelle x est compris entre 0,1 et 1,5 ; et dans laquelle y est compris entre >0 et 2, lesdits cristaux de BLT étant sous la forme de nanoparticules constituées d'un ou plusieurs desdits cristaux, au moins 50 % du nombre total desdites nanoparticules présentant au moins une dimension allant jusqu'à 200 nm.

2. Composition selon l'une quelconque de la revendication 1, x étant compris entre 0,5 et 1,0, de manière appropriée x étant compris entre 0,7 et 0,8.

3. Composition selon l'une quelconque de la revendication 1 ou la revendication 2, y étant compris entre 0,01 et 1,8.

**4.** Composition selon l'une quelconque de la revendication 1 à la revendication 3, le rapport molaire du Fe au Ti étant choisi parmi (0,0625 et 2,9375), (0,125 et 2,875), (0,25 et 2,75), (1 et 2) et (1,5 et 1,5).

**5.** Composition selon l'une quelconque de la revendication 1 à la revendication 4, au moins 90 % du nombre total desdites nanoparticules ayant au moins une dimension allant jusqu'à environ 200 nm, jusqu'à environ 150 nm, ou jusqu'à environ 100 nm, lesdites nanoparticules étant constituées éventuellement de cristaux ayant la même formule chimique.

**6.** Composition selon l'une quelconque de la revendication 1 à la revendication 5, lesdits un ou plusieurs cristaux de BLT étant présents dans la composition sous la forme de nanoparticules à une concentration comprise dans la plage allant d'environ 0,001 % en poids à environ 40 % en poids de la composition totale.

**7.** Composition selon l'une quelconque de la revendication 1 à la revendication 6, lesdites nanoparticules desdits un ou plusieurs cristaux de BLT étant dispersées avec un dispersant dans une matrice polymère comprenant en outre un polymère thermoplastique gonflé d'huile.

**8.** Composition selon la revendication 7, ladite matrice polymère étant sous la forme de flocons de matrice polymère, chaque flocon desdits flocons de matrice de polymère présentant une longueur de flocons (Lf), une largeur de flocon (Wf) et une épaisseur de flocon (Tf), lesdits flocons de matrice polymère présentant un rapport de forme de flocon sans dimension (Rf) défini par : Rf = (Lf•Wf)/(Tf)$^2$, par rapport à un groupe représentatif d'au moins dix flocons de matrice polymère un Rf moyen étant d'au moins 5 ; et lesdites nanoparticules dans ledit groupe représentatif présentant une taille de particule moyenne (D50) d'au plus 100 nm.

**9.** Composition selon l'une quelconque de la revendication 7 ou la revendication 8, ledit dispersant conçu pour disperser les nanoparticules de cristaux de BLT dans ladite matrice polymère ayant une valeur d'équilibre hydrophile-lipophile (HLB) d'au plus de 9, généralement d'au plus de 6, de manière appropriée d'au plus de 4, et de manière plus appropriée d'au plus de 3.

**10.** Composition selon l'une quelconque de la revendication 7 à la revendication 9, ledit polymère thermoplastique dans la matrice polymère comprenant au moins un polymère éthylène-acrylique (EAA), un polymère éthylène-méthacrylique (EMMA), un polymère acétate d'éthylvinyle (EVA), ou une combinaison de ceux-ci.

**11.** Composition selon l'une quelconque de la revendication 1 à la revendication 10, ledit spectre d'absorption de la composition présentant une longueur d'onde critique d'au moins 370 nm.

**12.** Composition selon l'une quelconque de la revendication 1 à la revendication 11, ladite aire sous la courbe (AUC) formée par l'absorption des UV desdits un ou plusieurs cristaux de BLT en fonction d'une longueur d'onde comprise dans la plage de 280 nm à 400 nm (AUC$_{280\text{-}400}$) d'au moins 75 %, d'au moins 85 % ou d'au moins 95 % de l'AUC formée par les mêmes cristaux à la même concentration dans la plage de 280 nm à 700 nm (AUC$_{280\text{-}700}$).

**13.** Composition selon l'une quelconque de la revendication 1 à la revendication 12, formulée comme l'une des compositions suivantes : (a) une composition dermatologique destinée à une application sur la peau des humains et des animaux non humains ; (b) une composition de traitement capillaire destinée à une application sur les cheveux des humains et les poils des animaux non humains ; ou (c) une composition de revêtement destinée à une application sur une surface inanimée.

**14.** Utilisation de la composition selon l'une quelconque de la revendication 1 à la revendication 13, pour la protection des cheveux/poils d'un sujet ou pour protéger un objet inanimé contre un effet nuisible du rayonnement ultraviolet, en particulier la protection contre le rayonnement ultraviolet A et le rayonnement ultraviolet B.

**15.** Composition selon l'une quelconque de la revendication 1 à la revendication 13 pour une utilisation dans la protection de la peau d'un sujet contre un effet nuisible du rayonnement ultraviolet, en particulier la protection contre le rayonnement ultraviolet A et le rayonnement ultraviolet B.

[117]

**FIG. 1**

**FIG. 2**

**FIG. 3**

**FIG. 4**

**FIG. 5**

**FIG. 6**

| HV | HFW | mag ☐ | WD | det | curr | —————— 100 nm —————— |
| 30.00 kV | 373 nm | 800 000 x | 4.3 mm | STEM II | 0.40 nA | |

**FIG. 7A**

| HV | HFW | mag ☐ | WD | det | curr | —————— 100 nm —————— |
| 30.00 kV | 373 nm | 800 011 x | 4.3 mm | STEM II | 50 pA | |

**FIG. 7B**

**FIG. 8**

**FIG. 9**

**FIG. 10**

**FIG. 11**

**FIG. 12**

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016151537 A

- WO 2017013633 A

**Non-patent literature cited in the description**

- *CHEMICAL ABSTRACTS,* 1304-76-3
- *CHEMICAL ABSTRACTS,* 1309-37-1
- *CHEMICAL ABSTRACTS,* 1312-81-8
- *CHEMICAL ABSTRACTS,* 9003-04-7
- *CHEMICAL ABSTRACTS,* 13463-67-7